# EUROPEAN PATENT SPECIFICATION

(11) **EP 0 832 974 B1**
(45) Date of publication and mention of the grant of the patent: **02.04.2008**
(21) Application number: 97115801.9
(22) Date of filing: 11.09.1997
(51) Int. Cl.: C12N 15/53, C12N 9/04, C12N 1/21, C12N 5/10, C12P 7/24, C12P 7/26, C12P 7/40, C12P 17/00

(54) **Alcohol-aldehyd-dehydrogenases**
Alkohol-Aldehyd-Dehydrogenasen
Déshydrogénase d'alcool et d'aldéhyde

(30) Priority: 19.09.1996 EP 96115001
(43) Date of publication of application: 01.04.1998
(60) Divisional application: 06003507.8
(73) Proprietor: DSM IP Assets B.V., 6411 TE Heerlen (NL)
(72) Inventor: Asakura, Akira, Fujisawa-shi, Kanagawa-ken (JP); Hoshino, Tatsuo, Kamakura-shi, Kanagawa-ken (JP); Ojima, Setsuko, Fujisawa-shi, Kanagawa-ken (JP); Shinjoh, Masako, Kamakura-shi, Kanagawa-ken (JP); Tomiyama, Noribumi, Fujisawa-shi, Kanagawa-ken (JP)
(74) Representative: Seibel-Thomsen, Nadja

(56) References cited:
- EP-A- 0 448 969
- EP-A- 0 606 621
- STOORVOGEL J. ET AL.: "Characterization of the gene encoding quinohaemoprotein ethanol dehydrogenase of Comamonas testosteroni" EUR. J. BIOCHEM., vol. 235, 1996, page 690-698 XP002110757
- "Alcohol dehydrogenase complex structural gene-used in plasmid and enhancing efficiency of acetic acid fermentation for transformed acetic acid bacteria" GENESEQ DATABASE,16 April 1992, XP002110758
- TAMAKI T. ET AL.: "Cloning and sequencing of the gene cluster encoding two subunits of membrane-bound alcohol dehydrogenase from Acetobacter polyoxogenes" BIOCHIM. BIOPHYS. ACTA, vol. 1088, 1991, pages 292-300, XP002110759
- KONDO K. ET AL: "CHARACTERIZATION OF THE GENES ENCODING THE THREE-COMPONENT MEMBRANE-BOUND ALCOHOL DEHYDROGENASE FROM GLUCONOBACTER SUBOXYDANS AND THEIR EXPRESSION IN ACETOBACTER PASTEURIANUS" APPLIED AND ENVIRONMENTAL MICROBIOLOGY, vol. 63, no. 3, March 1997, pages 1131-1138, XP002914612
- REID M.F.: "Molecular Characterization of Microbial Alcohol Dehydrogenases" CRIT. REV. MICROBIOL., vol. 20, no. 1, 1994, page 13-56 XP002110760

## Description

The present invention relates to recombinant enzyme preparations of alcohol/aldehyde dehydrogenase(s) (hereinafter referred to as AADH or AADHs) having alcohol and/or aldehyde dehydrogenase activity (activities). The present invention also relates to novel recombinant DNA molecule(s) encoding AADH(s), recombinant expression vector(s) containing said DNA(s), and recombinant organism(s) containing said recombinant DNA molecule(s) and/or said recombinant expression vector(s). Furthermore, the present invention relates to a process for producing recombinant enzyme preparation(s) of AADH(s) and a process for producing aldehyde(s), carboxylic acid(s) and ketone(s), especially, 2-keto-L-gulonic acid (herein after referred to as 2KGA) by utilizing said recombinant enzyme preparation(s), and a process for producing aldehyde(s), carboxylic acid(s) and ketone(s), especially, 2KGA by utilizing said recombinant organism(s).

2-KGA is an important intermediate for the production of L-ascorbic acid (vitamin C). Numerous microorganisms are known to produce 2KGA from D-sorbitol or L-sorbose. Japanese Patent Publication No. 51-40154 (1976) discloses the production of 2KGA from D-sorbitot by microorganisms of the genus *Acetobacter, Bacterium* or *Pseudomonas.* According to Acta Microbiologica Sinica 21(2), 185 - 191 (1981), 2KGA can be produced from L-sorbose by a mixed culture of microorganisms, especially, *Pseudomonas striata* and *Gluconobacter oxydans.* European Patent Publication No. 0221 707 discloses the production of 2KGA from L-sorbose by *Pseudogluconobacter saccharoketogenes* with and without concomitant bacteria. European Patent Publication No. 0278 447 discloses a process for the production of 2KGA from L-sorbose by a mixed culture, which is composed of strain DSM No. 4025 *(Gluconobacter oxydans)* and DSM No. 4026 (*a Bacillus megaterium* strain). European Patent Publication No. 88116156 discloses a process for the production of 2KGA from L-sorbose by *Gluconobacter oxydans* DSM No. 4025.

From *G. oxydans* DSM No. 4025, AADH was purified and characterized to catalyte the oxidation of alcohols and aldehydes, and was thus capable of producing the corresponding aldehydes and ketones from alcohols, and carboxylic acids from aldehydes (seeEuropean Patent Publication No. 606621). More particularly, the AADH catalyzed the oxidation of L-sorbose to 2KGA via L-sorbosone. The physico-chemical properties of the purified sample of the AADH were as follows:
a) Optimum pH: about 7.0 - 9.0
b) Optimum temperature: about 20°C - 40°C
c) Molecular weight: 135,000 +/- 5,000 dalton (Consisting of two subunits in any combination of such α-subunit and β-subunit, each having a molecular weight of 64,500 +/- 2,000 and 62,500 +/- 2,000, respectively)
d) Substrate specificity: active on primary and secondary alcohols and aldehydes including L-sorbose, L-sorbosone, D-sorbitol, D-glucose, D-mannitol, D-fructose, DL-glycelaldehyde, ethanol, 1-propanol, 1-butanol, 1-pentanol, 1-hexanol, 1-heptanol, 2-propanol, 2-butanol, propionaldehyde, PEG 1000, PEG2000, PEG4000, PEG6000 and polyvinyl alcohol
e) Prosthetic group: pyrroloquinoline quinone
f) Isoelectric point: about 4.4

Once the gene(s) coding for said AADH have been cloned, they can be used for the construction of a recombinant organism capable of producing a large amount of the recombinant enzyme preparation of AADH or the various aldehydes, ketones and carboxylic acids, especially, 2KGA. However, there have been no reports so far of the cloning of such genes.

Therefore the present invention relates to novel recombinant enzyme preparation(s) of AADH(s) having alcohol and/or aldehyde dehydrogenase activity (activities). Comprised by the present invention are novel recombinant molecule(s) encoding the AADH(s); recombinant expression vector(s) containing said DNAs; recombinant organism(s) carrying said DNA(s) and/or recombinant expression vector(s); a process for producing the recombinant AADH(s); and a process for producing aldehyde(s), carboxylic acid(s) and ketone(s), especially, - 2KGA utilizing the recombinant AADH(s) or the recombinant organism(s).

More particularly, an aspect of the present invention concerns a recombinant enzyme preparation having an alcohol and/or aldehyde dehydrogenase activity which comprises enzymatic polypeptide identified by SEQ ID NO:5 or encoded by a nucleic acid which hybridizes under stringent hybridization and stringent washing conditions with SEQ ID NO : 1, said polypeptides having alcohol and/or aldehyde dehydrogenase activity. Polypeptides with at least 80% identity to SEQ ID NO: 5 include, but are not limited to, polypeptides identified by SEQ ID NO: 6, SEQ ID NO: 7, and SEQ ID NO: 8.

Another aspect of the present invention concerns a recombinant DNA molecule encoding an enzymatic polypeptide identified by SEQ ID NO:5 or a nucleic acid which hybridizes under stringent hybridization and stringent washing conditions with SEQ ID NO:1.

Furthermore the present invention is directed to DNA sequences encoding the polypeptides with alcohol and/or aldehyd dehydrogenase activity as disclosed e.g. in the sequence listing as well as their complementary strands, or those which include these sequences, DNA sequences which hybridize under standard conditions with such sequences or fragments thereof and DNA sequences, which because of the degeneration of the genetic code, do not hybridize under standard conditions with such sequences but which code for polypeptides having exactly the same amino acid sequence.

"Standard conditions" for hybridization mean in this context the conditions which are generally used by a man skilled in the art to detect specific hybridization signals and which are described, e.g. by Sambrook et al., "Molecular Cloning" second edition, Cold Spring Harbour Laboratory Press 1989, New York, or preferably so called stringent hybridization and non-stringent washing conditions or more preferably so called stringent hybridization and stringent washing conditions a man skilled in the art is familiar with and which are described, e.g. in Sambrook et al. (s.a.). Furthermore DNA sequences which can be made by the polymerase chain reaction by using primers designed on the basis of the DNA sequences disclosed herein by methods known in the art are also an object of the present invention. It is understood that the DNA sequences of the present invention can also be made synthetically as described, e.g. in EP 747 483.

Further aspects of the present invention concern a recombinant expression vector which carries one or more of the recombinant DNA molecule(s) defined above and a recombinant organism which carries the recombinant expression vector defined above and/or carries one or more recombinant DNA molecule(s) on a chromosome.

A further aspect of the present invention concerns a process for producing a recombinant enzyme preparation having an alcohol and/or aldehyde dehydrogenase activity as defined above, which comprises cultivating a recombinant organism defined above in an appropriate culture medium and recovering said recombinant enzyme preparation.

Another aspect of the present invention concerns a process for producing an aldehyde, ketone or carboxylic acid product from a corresponding substrate which comprises converting said substrate into the product by the use of a recombinant organism as defined above.

Moreover another aspect of the present invention concerns a process for producing 2-keto-L-gulonic acid which comprises the fermentation of a recombinant organism as defined above in an appropriate medium containing L-sorbose and/or D-sorbitol.

Another aspect of the present invention concerns a process for producing an aldehyde, ketone or carboxylic acid product from a corresponding substrate which comprises the incubation of a reaction mixture containing a recombinant enzyme preparation of the present invention.

Further more another aspect of the present invention concerns a process for producing 2-keto-L-gulonic acid which comprises the incubation of a reaction mixture containing a recombinant enzyme preparation defined above and L-sorbose and/or D-sorbitol.

It is also an object of the present invention to provide a process for the production of vitamin C from 2-keto-L-gulonic acid characterized therein that a process for the production of 2-keto-L-gulonic acid as described above is effected and the 2-keto-L-gulonic acid obtained by such process is transformed into vitamin C (L-ascorbic acid) by methods known in the art.

Before describing the present invention in more detail a short explanation of the attached figures is given.
Figure 1 schematically illustrates the structures of the recombinant expression vectors each carrying the recombinant DNA. molecule which encodes the recombinant Enzyme A or B of the present invention.
Figure 5 shows the alignment of the amino acid sequences of the mature Enzyme A and Enzyme B.
Figure 7 shows the restriction map of the genes of Enzymes A and B.
Figure 9 shows a site-directed mutagenesis to introduce a *Bam*HI site upstream of the Enzyme B gene.
Figure 10 illustrate a scheme of the replacement of the promoter for the Enzyme B gene.

The AADH genes of the present invention encode the AADH enzymes capable of catalyzing the oxidation of various alcohols and aldehydes as described above. Specifically speaking the particular genes of AADHs present in *Gluconobacter* were cloned and expressed. Alternative sources in addition to *Gluconobacter* may well be found among the other organisms by the man skilled in the art using the teachings of the present invention.

A specific and preferred *Gluconobacter oxydans* strain has been deposited at the Deutsche Sammlung von Mikroorganismen in Göttingen (Germany) under DSM No. 4025.

Moreover, a subculture of the strain has also been deposited in the Agency of Industrial Science and Technology, Fermentation Research Institute, Japan, under the deposit No.: FERM BP-3812. European Patent publication No. 0278 477 disclose the characteristics of this strain.

The AADH genes and the recombinant microorganisms utilized in the present invention can be obtained by the following steps:
(1) Cloning the AADH genes from a chromosomal DNA by colony- or plaque-hybridization, PCR cloning, Western-blot analysis, Southern-blot hybridization and the like.
(2) Determining the nucleotide sequences of such AADH genes by usual methods and constructing recombinant expression vectors which contain and express AADH genes efficiently.
(3) Constructing recombinant microorganisms carrying recombinant AADH genes on recombinant expression vectors or on chromosomes by transformation, transduction, transconjugation and electroporation.

The materials and the techniques applicable to the above aspect of the present invention are exemplified in details as described in the following:

A total chromosomal DNA can be purified by a procedure well known in the art (Marmur J., J. Mol. Biol. 3:208, 1961). Then, a genomic library of the strain for such genes can be constructed with the chromosomal DNA and the vectors described below in detail. The genes encoding AADHs can be cloned in either plasmid or phage vectors from the total chromosomal DNA by the following methods:
(i) determining the partial amino acid sequences of the purified enzyme, according to the sequence information, synthesizing the oligonucleotides, and selecting the objective gene from the gene library by Southern-blot-, colony-, or plaque-hybridization;
(ii) by amplifying the partial sequence of the desired gene by polymerase chain reaction (PCR) with the oligonucleotides synthesized as described above as the primers and with the PCR product as a probe, selecting the complete sequence of the objective gene from the gene library by Southern-blot-, colony-, or plaque-hybridization;
(iii) by preparing the antibody reacting against the desired enzyme protein by such a method as previously described, e.g. in Methods in Enzymology, vol. 73, p 46, 1981, and selecting the clone which expresses the desired polypeptide by immnunological analysis including Western-blot analysis;
(iv) by aligning the amino acid sequences of the homologs to the one of the desired enzyme, selecting the amino acid sequences which are well conserved, synthesizing the oligonucleotides encoding the conserved sequences, amplifying the partial sequence of the desired gene by PCR with the above oligonucleotides as the primers, and selecting the complete sequence as described above (ii).

The nucleotide sequence of the desired gene can be determined by a well known method such as the dideoxy chain termination method with the M13 phage (Sanger F., et al., Proc. Natl. Acad. Sci.USA, 74:5463-5467, 1977).

By using the information of the so determined nucleotide sequence (in consideration of the codon usage) a gene encoding evolutionally divergent alcohol and/or aldehyde dehydrogenases, can be isolated from a different organism by colony- or Southern-hybridization with a probe synthesized according to the amino acid sequence deduced from said nucleotide sequence or by the polymerase chain reaction with primers also synthesized according to said information, if necessary.

To express the desired gene or generally speaking the desired DNA sequence of the present invention efficiently, various promoters can be used; for example, the original promoter of said gene, promoters of antibiotic resistance genes such as the kanamycin resistant gene of Tn5 (Berg, D. E., and C. M. Berg. 1983. Bio/Technology 1:417-435), the ampicillin resistant gene of pBR322, a promoter of the beta-galactosidase gene of *Escherichia coli* (lac), trp-, tac- trc-promoter, promoters of lambda phages and any promoters which can be functional in the hosts consisting of microorganisms including bacteria such as *E. coli, P. putida, Acetobacter xylinum, A. pasteurianus, A. aceti, A. hansenii* and *G. oxydans,* mammalian and plant cells.

Furthermore other regulatory elements, such as a Shine-Dalgarno (SD) sequence (for example, AGGAGG etc. including natural and synthetic sequences operable in the host cell) and a transcriptional terminator (inverted repeat structure including any natural and synthetic sequence operable in the host cell) which are operable in the host cell into which the coding sequence will be introduced can be used with the above described promoters.

For the expression of periplasmic polypeptides (AADHs) a signal peptide, which contains usually 15 to 50 amino acid residues totally hydrophobic, is indispensable. A DNA encoding a signal peptide can be selected from any natural or synthetic sequence operable in the host cell.

A wide variety of host/cloning vector combinations may be employed in cloning the double-stranded DNA. Cloning vector is generally a plasmid or phage which contains a replication origin, regulatory elemtents, a cloning site including a multi-cloning site and selection markers such as antibiotic resistance genes including resistance genes for ampicillin, tetracycline, kanamycin, streptomycin, gentamicin, spectinomycin etc.

Preferred vectors for the expression of the DNA sequences of the present invention in *E. coli* are selected from any vectors usually used in *E. coli,* such as pBR322 or its derivatives including pUC18 and pBluescript II, pACYC177 and pACYC184 (J. Bacteriol., 134:1141-1156, 1978) and their derivatives, and a vector derived from a broad host range plasmid such as RK2 and RSF1010. A preferred vector for the expression of the DNA sequences of the present invention in *Gluconobacter* including *G. oxydans* DSM No. 4025 and *P. putida* is selected from any vectors which can replicate in *Gluconobacter* and/or *P. putida,* as well as a preferred cloning organism such as in *E. coli.* The preferred vector is a broad-host-range vector such as a cosmid vector like pVK102 and its derivatives and RSF1010 and its derivatives, and a vector containing a replication origin functional in *Gluconobacter* and another origin functional in *E. coli.* Copy number and stability of the vector should be carefully considered for stable and efficient expression of the cloned gene and also for efficient cultivation of the host cell carrying the cloned gene. DNA molecules containing transposable elements such as Tn5 can be also used as a vector to introduce the DNA sequence of the present invention into the preferred host, especially on a chromosome. DNA molecules containing any DNAs isolated from the preferred host together with the desired DNA sequence of the present invention are also useful to introduce the desired DNA sequence of the present invention into the preferred host, especially on a chromosome. Such DNA molecules can be transferred to the preferred host by transformation, transduction, transconjugation or electroporation.

Useful hosts may include microorganisms, mammalian cells, and plant cells and the like. As preferable microorganisms, there may be mentioned bacteria such as *E*. *coli, P.* putida, *A. xylinum, A. pasteurianus, A. aceti, A. hansenii, G. oxydans,* and any Gram-negative bacteria which are capable of producing recombinant AADHs. Functional equivalents, subcultures, mutants and variants of said microorganism can be also used in the present invention. A preferred strain is *E. coli* K12 and its derivatives, *P. putida* or *G. oxydans* DSM No. 4025.

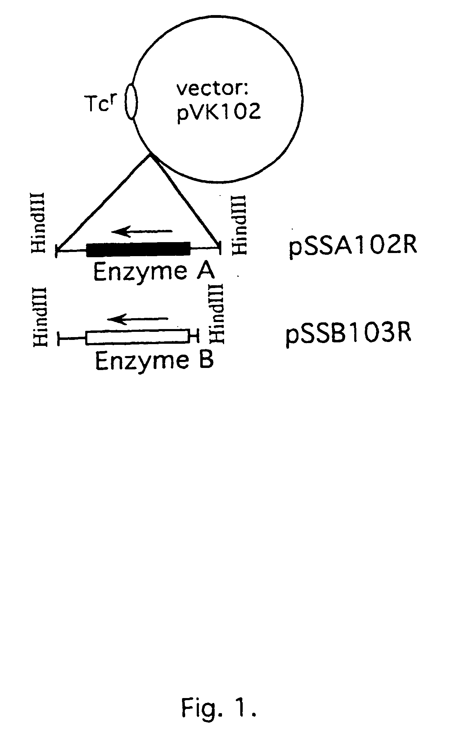

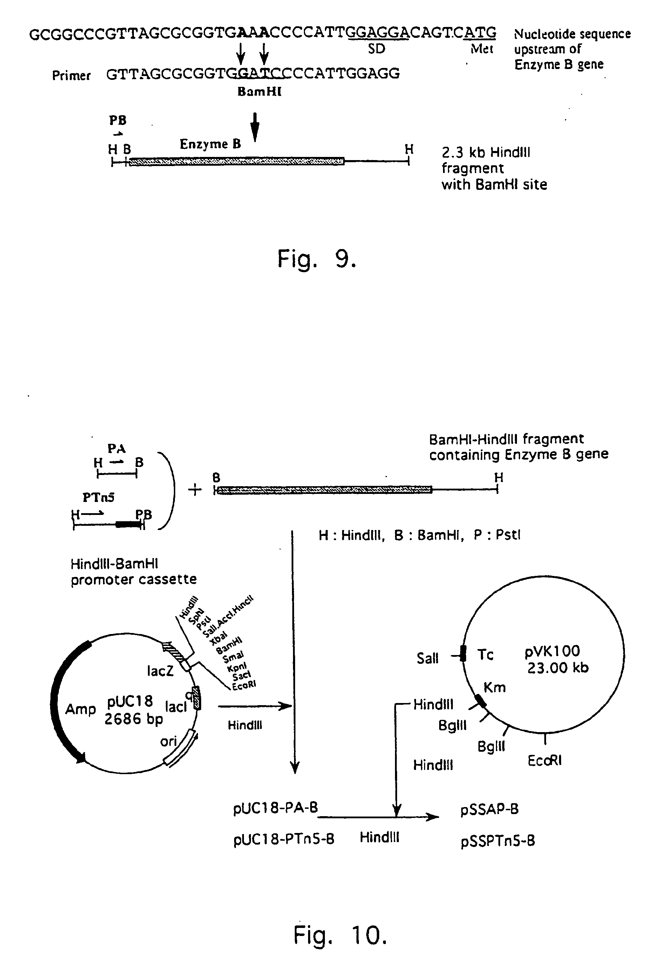

The functional AADH encoding DNA sequence of the present invention is ligated into a suitable vector containing a regulatory region such as a promoter and a ribosomal binding site operable in the host cell described above using well-known methods in the art to produce an expression plasmid. Structures of such recombinant expression vectors are specifically shown in Fig. 1 and 10.

To construct a recombinant microorganism carrying a recombinant expression vector, various gene transfer methods including transformation, transduction, conjugal mating (Chapters 14 and 15, Methods for general and molecular bacteriology, Philipp Gerhardt et al. ed., American Society for Microbiology, (1994), and electroporation can be used. The method for constructing a recombinant organism may be selected from the methods well-known in the field of molecular biology. Usual transformation systems can be used for *E. coli, Pseudomonas* and *Acetobacter.* A transduction system can also be used for *E. coli.* Conjugal mating systems can be widely used in Gram-positive and Gram-negative bacteria including *E. coli, P. putida* and G. *oxydans.* A preferred conjugal mating method is described in WO89/06688. The conjugation can occur in liquid media or on a solid surface. The preferred recipient is selected from *E. coli, P.* putida and *G. oxydans* which can produce active AADHs with a suitable recombinant expression vector. The preferred recipient for 2KGA production is G. *oxydans* DSM No. 4025. To the recipient for conjugal mating, a selective marker is usually added; for example, resistance against nalidixic acid or rifampicin is usually selected.

The AADHs provided by the present invention catalyze the oxidation of alcohols and/or aldehydes, and are thus capable of producing aldehydes, ketones or carboxylic acids from the corresponding substrates. More particularly, the AADHs provided by the present invention can catalyze the oxidation of L-sorbose to 2KGA via L-sorbosone and/or the oxidation of D-sorbitol to L-sorbose. More particularly, the AADHs provided by the present invention contain Enzyme A, which has the amino acid sequence shown in SEQ ID NO:5.

Furthermore, the AADHs of the present invention contain enzymes which or encoded by a nucleic acid which hybridizes under stringent hybridization and stringent washing conditions with SEQ ID NO : 1, said enzymes having alcohol and/or aldehyde dehydrogenase activity.

Enzyme A, A', A", and B genes, which have the nucleotide sequences shown in SEQ ID NO. 1, 2, 3, and 4, respectively, and encode the polypeptides having the amino acid sequences shown in SEQ ID NO. 5, 6, 7, and 8, respectively can be derived from *G. oxydans.* strain DSM No. 4025.

The AADHs including Enzymes A, A', A" and B provided by the present invention can be prepared independently by cultivating an appropriate organism, disrupting the cells and isolating and purifying them from cell free extracts of disrupted cells, preferably from the soluble fraction of the microorganism.

The recombinant organisms provided in the present invention may be cultured in an aqueous medium supplemented with appropriate nutrients under aerobic conditions. The cultivation may be conducted at a pH between about 4.0 and 9.0, preferably between about 6.0 and 8.0. While the cultivation period varies depending upon pH, temperature and nutrient medium used, usually 2 to 5 days will bring about favorable results. A preferred temperature range for carrying out the cultivation is from about 13°C to 45°C preferably from about 18°C to 42°C.

It is usually required that the culture medium contains such nutrients as assimilable carbon sources, digestible nitrogen sources and inorganic substances, vitamins, trace elements and other growth promoting factors. As assimilable carbon sources, glycerol, D-glucose, D-mannitol, D-fructose, D-arabitol, D-sorbitol, L-sorbose, and the like can be used.

Various organic or inorganic substances may also be used as nitrogen sources, such as yeast extract, meat extract, peptone, casein, corn steep liquor, urea, amino acids, nitrates, ammonium salts and the like. As inorganic substances, magnesium sulfate, potassium phosphate, ferrous and ferric chlorides, calcium carbonate and the like may be used.

In the following, the properties of the purified recombinant AADH enzymes specifically from *P. putida* and the production method are summerized.

### (1) Enzyme activity

The AADHs of the present invention catalyze oxidation of alcohols and aldehydes including D-sorbitol, L-sorbose, and L-sorbosone in the presence of an electron acceptor according to the following reaction formula.
Alcohol+ Electron acceptor -> Aldehyde + Reduced electron acceptor
Alcohol+ Electron acceptor -> Ketone + Reduced electron acceptor
Aldehyde + Electron acceptor -> Carboxylic acid + Reduced acceptor
Sugar alcohol + Electron acceptor -> Aldose + Reduced electron acceptor
Sugar alcohol + Electron acceptor -> Ketose + Reduced electron acceptor
Aldehyde ketose+Electron acceptor -> Ketocarboxylic acid+Reduced electron acceptor
Carboxylic acid+Electron acceptor -> Ketocarboxylic acid+Reduced electron acceptor

The enzymes do not utilize molecular oxygen as an acceptor. As an acceptor, 2,6-dichlorophenolindophenol (DCIP), phenazine methosulphate (PMS), Wurster's blue, ferricyanide, coenzyme Q or cytochrome c can be used.

One unit of enzyme activity was defined as the amount of enzyme which catalyzed the reduction of 1 µmole of DCIP per minute. The extinction coefficient of DCIP at pH 8.0 was taken as 15 mM⁻¹. The standard reaction mixture (1.0 ml) contained 0.1 mM DCIP, 1 mM PMS, 2 to 125 mM substrate, 50 mM Tris-malate-NaOH buffer (pH 8.0), and 10 µl of the enzyme solution. A reference cuvette contained all the above components except the substrate.

### (2) Properties of the AADHs

### a) Substrate specificity and products of the enzymatic reaction

The Enzymes A, A', A" and B were characterized by their substrate specificities as described above using 8 substrates: n-propanol, isopropanol, D-glucose, D-sorbitol, L-sorbosone, D-mannitol, L-sorbose, and D-fructose. The results are indicated in Table 1.

**Table 1. Substrate specificity of the Enzymes A, A', A" and B**

| (units/mg of purified protein) | | | | |
|---|---|---|---|---|
| Substrate | Enzyme A | Enzyme A'* | Enzyme A" | Enzyme B |
| 50 mM n-Propanol | 139.6 | 180.7 | 262.3 | 40.0 |
| 50 mM Isopropanol | 76.8 | 108.9 | 154.9 | 72.3 |
| 50 mM D-Glucose | 2.4 | 0.0 | 17.8 | 943.9 |
| 125 mM D-Sorbitol | 14.0 | 7.8 | 30.1 | 130.9 |
| 2mM L-Sorbosone | 23.15 | 5.0 | 26.5 | 73.6 |
| 50 mM D-Mannitol | 7.1 | 1.3 | 6.2 | 517.4 |
| 125 mM L-Sorbose | 47.4 | 1.6 | 30.3 | 8.4 |
| 125 mM D-Fructose | 30.7 | 2.9 | 17.3 | 2.1 |

| | | | | |
|---|---|---|---|---|
| *: Values of the Enzyme A' was corrected by 1.5-fold, since purity of the enzyme was about 65%. | | | | |

Enzyme B showed a high reactivity for D-glucose or D-mannitol, but relatively low reactivity for n-propanol and isopropanol. Enzyme A, Enzyme A' and Enzyme A" showed a high reactivity for n-propanol and isopropanol, but a low reactivity for D-glucose and D-mannitol; the enzymes showed similar substrate specificity patterns, except that the Enzyme A' had a very low reactivity for L-sorbose or D-fructose.

Product(s) formed from a substrate in the reaction with Enzyme A, Enzyme A', Enzyme A" or Enzyme B was analyzed by thin layer chromatography (TLC) and/or high performance liquid chromatography (HPLC) with authentic compounds. Enzyme A, Enzyme A' and Enzyme A" (designated A group) converted D-sorbitol, L-sorbose, L-sorbosone, D-mannitol, and D-fructose to D-glucose with L-gulose, L-sorbosone with 2KGA, 2KGA, D-mannose, and 2-keto-D-gluconic acid (2KD), respectively. Enzyme B (designated B group) converted D-glucose, D-sorbitol, L-sorbosone, D-mannitol, L-idose, glycerol, D-gluconic acid, D-mannoic acid to D-gluconate, L-sorbose, 2KGA, D-fructose, L-idonic acid, dihydroxyacetone, 5-keto-D-gluconic acid, and 5-keto-D-mannoic acid, respectively. In the analogy to the reactivity for L-sorbosone, D-glucosone can be converted to 2KD by all of above mentioned AADHs; actually A group enzymes produced, 2KD from D-fructose whose possible direct product is D-glucosone. All of the enzymes showed the activity for alcohols including sugar alcohol such as D-sorbitol and D-mannitol, and aldehydes including aldose such as D-glucose and ketose such as L-sorbosone.

### b) Optimum pH

All the enzymes have their optimal point at pH 8.0 - 8.5 as shown in Table 2. The Enzymes A" and B have a relatively wide pH range toward a lower pH, compared with the Enzymes A and A'.

**Table 2. Optimal pH of the enzymes**

| (Relative activity, %) | | | | |
|---|---|---|---|---|
| pH | Enzyme A | Enzyme A' | Enzyme A" | Enzyme B |
| 6.0 | 6.5 | 2.1 | 35.0 | 21.0 |
| 6.5 | 13.0 | 9.3 | 57.3 | 51.6 |
| 7.0 | 33.1 | 22.5 | 74.8 | 61.6 |
| 7.5 | 57.7 | 46.8 | 90.0 | 75.3 |
| 8.0 | 100.0 | 100.0 | 100.0 | 100.0 |
| 8.5 | 113.2 | 142.7 | 85.6 | 62.2 |
| 9.0 | 50.0 | 2.1 | 46.5 | 8.0 |
| 9.5 | 19.6 | 1.8 | 23.9 | 0.0 |

### c) pH stability

Enzymes A, A', A" and B were incubated in buffers of various pH-values for 3 hours at 25 °C and the residual activities were assayed and expressed as relative values against that obtained by no incubation at pH8. Enzymes A, A', A" and B were stable between pH 6 to 9 as shown in Table 3.

**Table 3. pH stability of the enzymes**

| (Relative activity, %) | | | | |
|---|---|---|---|---|
| pH | Enzyme A | Enzyme A' | Enzyme A" | Enzyme B |
| 4.0 | 5.4 | 0.0 | 6.2 | 25.2 |
| 5.0 | 32.0 | 10.0 | 77.9 | 56.1 |
| 6.0 | 74.7 | 82.7 | 105.8 | 100.9 |
| 7.0 | 76.9 | 96.9 | 100.9 | 101.9 |
| 8.0 | 80.1 | 100.0 | 99.0 | 114.0 |
| 9.0 | 60.1 | 97.3 | 100.9 | 101.9 |
| 10.0 | 53.2 | 85.4 | 104.0 | 85.5 |
| 11.0 | 31.0 | 61.3 | 79.2 | 70.1 |

### d) Thermal stability

The residual activities after the treatment of the enzymes at 4, 20, 30, 40, 50, and 60°C for 5 minutes are shown in Table 4.

**Table 4. Thermal stability of the enzymes**

| (Relative activity, %) | | | | |
|---|---|---|---|---|
| Temperature | Enzyme A | EnzymeA' | Enzyme A" | Enzyme B |
| 4 °C | 100.0 | 100.0 | 100.0 | 100.0 |
| 20 °C | 91.5 | 100.8 | 96.0 | 97.2 |
| 30 °C | 78.0 | 103.6 | 86.1 | 95.4 |
| 40 °C | 19.9 | 78.9 | 72.8 | 84.6 |
| 50 °C | 4.1 | 0.6 | 26.6 | 29.2 |
| 60 °C | 2.9 | 0.0 | 13.3 | 0.0 |

### e) Effect of metal ions and inhibitors

Remaining activities after the treatment of the enzymes with various metals and inhibitors are shown in Table 5. MgCl₂ and CaCl₂ were nearly inert to the enzymes, while the other metal ions, especially CuCl₂, significantly affected the reactivity. EGTA and EDTA inhibited the Enzymes A, A' and A", remarkably. However, Enzyme B was less inhibited than the A group enzymes by EDTA and EGTA.

**Table 5. Effect of metals and inhibitors on activities of the Enzymes A, A', A" and B.**

| (Relative remaining activity) | | | | | |
|---|---|---|---|---|---|
| Compound | Enzyme | A | A' | A" | B |
| | Substrate | L-Sorbose | n-Propanol | L-Sorbose | D-Sorbitol |
| 5mM CoCl₂ | | 16.6 | 7.9 | 46.9 | 23.6 |
| 5mM CuCl₂ | | 0.0 | 0.0 | 0.0 | 0.0 |
| 5mM ZnCl₂ | | 1.5 | 6.1 | 19.2 | 0.0 |
| 5mM MgCl₂ | | 96.3 | 85.3 | 78.8 | 100.0 |
| 5mM CaCl₂ | | 98.8 | 95.3 | 123.0 | 102.9 |
| 5mM MnCl₂ | | 0.0 | 45.7 | 0.0 | 0.0 |
| 5mM FeCl₂ | | 16.6 | 0.0 | 0.0 | 5.9 |
| 5mM FeCl₃ | | 7.8 | 0.0 | 44.7 | 0.0 |
| 5mM NiSO₄ | | 42.7 | 59.7 | 90.3 | 79.4 |
| 10mM EDTA | | 43.1 | 55.1 | 52.6 | 91.3 |
| 10mM EGTA | | 20.4 | 16.7 | 56.4 | 74.0 |
| 1mM NaF | | 98.2 | 97.1 | 94.9 | 100.8 |
| 2mM NEM | | 91.7 | 97.2 | 94.9 | 100.8 |
| 1mM ICH₂COONa | | 97.2 | 78.3 | 95.3 | 100.2 |
| 0.5mM Hydroxylamine-HCl | | 104.6 | 98.8 | 97.2 | 102.1 |

### f) Molecular weight and subunit

Enzymes A, A', A" and B purified from *P. putida* transconjugants consist of one type of unit with the molecular weight of about 64,000, 62,500, 62,500 and 60,000, respectively, as measured by sodium dodecyl sulfate polyacrylamide gel electrophoresis. They can be heterodimers consisting of any two units of Enzymes A, A', A" and B when Enzymes A, A', A" and B genes/DNA sequences are expressed in the same host.

### g) N-terminal amino acid sequence

N-terminal sequences of mature Enzymes A and B are
Enzyme A : Gln-Val-Thr-Pro-Val-Thr----
Enzyme A" : Blocked N-terminal residue
Enzyme B Gln-Val-Thr-Pro-Ile-Thr-Asp-Glu-Leu-Leu- Ala----.
N-terminus of the mature Enzyme A' is not determined because of an insufficient purity of the sample.

### (3) Production of the AADHs

Cells are harvested from the fermentation broth by centrifugation or filtration. The cells are suspended in the buffer solution and disrupted by means of a homogenizer, sonicator or treatment with lysozyme and the like to give a disrupted solution of cells.

AADHs are isolated and purified from a cell free extract of disrupted cells, preferably from the soluble fraction of the microorganisms by usual protein purification methods such as ammonium sulfate precipitation, dialysis, ion exchange chromatographies, gel filtration chromatographies, and affinity chromatographies.

### (4) Enzyme reaction

Enzyme reaction was performed at pH values from about 6.0 to about 9.0 at the temperature of about 10°C to about 50°C, preferrably of 20°C to 40°C in the presence of an electron acceptor, for example, DCIP, PMS, Wurster's blue, ferricyanide, coenzyme Q, cytochrome c and the like in a buffer such as Tris-HCl buffer, phosphate buffer and the like. The concentration of the substrate in a reaction mixture can vary depending on the other reaction conditions but, in general, is desirable to be about 1 - 200 g/l, most preferably from 1 - 100 g/l.

In the enzyme reaction, AADHs may also be used in an immobilized state with an appropriate carrier. Any means of immobilizing enzymes generally known to the art may be used. For instance, the enzyme may be bound directly to membrane granules or the like of a resin having functional group(s), or it may be bound through bridging compounds having functional group(s), for example, glutaraldehyde, to the resin.

The enzymatic polypeptides of the present invention are usually produced in the form of dimers. Such dimers contain homodimers of Enzyme A. Thus the recombinant enzyme preparation of the present invention also contain one or more of said homodimers or of enzymes or encoded by a nucleic acid which hybridizes under stringent hybridization and stringent washing conditions with SEQ ID NO : 1, said enzymes having alcohol and/or aldehyde dehydrogenase activity. These enzymes include, but are not limited to, Enzyme A', Enzyme A", and Enzyme B, which have the amino acid sequence shown in SEQ ID NO: 6, 7, and 8, respectively.

The recombinant organisms provided by the present invention are highly useful for the production of the recombinant enzyme preparations of AADHs having an alcohol and/or aldehyde dehydrogenase activity. Said organisms are also useful for the production of aldehydes, carboxylic acids and ketones, especially, 2KGA by utilizing said recombinant enzyme preparations, and by utilizing said recombinant organisms.

The production of 2KGA with the said recombinant organisms can be performed in the fermentation with the medium and culture conditions as described above. The production of 2KGA may be performed with the recombinant organisms described above together with the concomitant organisms such as *E*. *coli, P. putida* and *Bacillus megaterium.*

### Examples

### Example 1. Cloning of AADH genes

### (1) Construction of a genomic library of G. oxydans DSM No. 4025

Chromosomal DNA was prepared as follows. *G. oxydans* DSM No. 4025 was cultivated on an agar plate containing 20 ml of NS2 medium consisting of 5.0% D-mannitol, 0.25% MgSO₄·7H₂O, 1.75% corn steep liquor, 5.0% baker's yeast (Oriental Yeast Co., Osaka, Japan), 0.5% CaCO₃, 0.5% urea (sterilized separately) and 2.0% agar (pH 7.0 before sterilization) at 27°C for 3 days. The cells were collected from the agar plate, washed with 10 ml of 10 mM Tris-HCl buffer (pH 8.0) containing 1mM EDTA and resuspended in 5 ml of 10 mM Tris-HCl buffer (pH 8.0) containing 20 mM EDTA. The cell suspension was treated with lysozyme (Sigma Chemicals Co., St. Louis, Mo., USA) at a final concentration of 400 µg/ml at 37°C for 30 minutes, then with pronase (400 units) at 37°C 30 minutes and with 1% SDS at 37°C for 1 hour. Chromosomal DNA was treated with phenol and RNase A (Boheringer Mannheim, GmbH, Mannheim, Germany) according to the method described by Maniatis et al. (Molecular cloning: a laboratory manual. Cold Spring Harbor Laboratory, Cold Spring Harbor, N. Y., (1982). Chromosomal DNA (200 µg) was digested with 168 units of *Sal*I (Boehringer Mannheim) at 37°C for 5 to 90 minutes. The resulting partially digested fragments of 15 - 35 kb were isolated by preparative agarose gel electrophoresis (agarose: 0.7%); the gel piece containing the desired fragments was cut out and the DNAs were electro-eluted from the gel into TAE buffer consisting of 40 mM Tris-acetate and 2 mM EDTA. Thus, 40 µg of the DNAs were obtained. In parallel, 8 µg of the cosmid vector pVK102 (ATCC 37158) was completely digested with *Sal*I and treated with calf intestine alkaline phosphatase (Boehringer Mannheim) according to the supplier's recommendation. pVK102 (0.4 µg) was ligated with the 15-35 kb *Sal*I fragments (0.2 - 2 µg) by the ligation kit (Takara Shuzo Co. Ltd., Kyoto, Japan) at 26°C for 10 minutes. The ligated DNAs were then used for in vitro packaging according to the method described by the supplier (Amersham): mixing the ligated DNAs with the phage coat protein parts. The resulting phage particles were used to infect *E. coli* ED8767 (Murray, N. E., W. J. Brammar and K. Murray. Mol. Gen. Genet., 150:53-61, 1977). About 3,000 Km^{r} Tc^{s} colonies were obtained and all of the colonies tested (24 colonies) possessed the insert DNAs; its average size was 26.5 kb. Another cosmid library of *G. oxydans* DSM No. 4025 containing 55,000 clones was constructed by using chromosomal DNA of *G. oxydans* DSM No. 4025 partially digested with *Eco*RI and inserting them into the*Eco*RI site of pVK100 by almost the same method described above. All of the colonies tested (24 colonies) possessed insert DNAs (average size; 27 kb).

These two cosmid libraries in E. *coli* ED8767 were then transferred into *E. coli* S 17-1 (Tra⁺, Bio/Technology, 1:784-791, 1983) by using the mixture of recombinant plasmid DNAs extracted from E. *coli* ED8767 libraries. About 4,000 Km^{r} transformants of *E. coli* S17-1 were picked up, cultivated individually in microtiter plates containing 100 µl of LB consisting of 10 g/l of Bactotrypton (Difco), 5 g/l of yeast extract (Difco) and 5 g/l of NaCl supplemented with 50 µg/ml kanamycin at 37°C, and stocked with 15% glycerol at -80°C as cosmid libraries in E. *coli* S17-1.

The G. *oxydans* DSM No. 4025-*Sal*I and *-Eco*RI cosmid libraries were constructed in *E. coli* S17-1. From the library, 1,400 clones were individually transferred from *E. coli* S17-1 into *P. putida* ATCC 21812 by conjugal mating. 1,400 cultures stocked in microtiter plates at -80°C were thawed and transferred to microtiter plates containing 100 µl of fresh LB medium in each well with a plate transfer cartridge (Nunc) and cultivated at 37°C overnight. Nalidixic acid resistant (Nal^{r}) *P. putida* ATCC21812 was cultivated at 30°C overnight in 100 ml of MB medium consisting of 2.5% mannitol, 0.5% yeast extract (Difco Laboratories, Detroit, Mich.) and 0.3% Bactotryptone (Difco). Fifty µl of the *P. putida* culture was individually added to the 1,400 wells containing cultures of the cosmid library. The 1,400 cell mixtures were spotted with plate transfer cartridges onto nitrocellulose filters placed on the surface of FB agar medium consisting of 5% fructose, 1% yeast extract (Difco), 1% polypeptone (Daigo Eiyo, Japan) and 1.8% agar and cultivated at 27°C overnight. Nalidixic acid was used for the counter-selection of transconjugants against donor *E. coli.* The cells grown on the filters were individually streaked onto MB agar medium containing 50 µg/ml of nalidixic acid and 50 µg/ml of kanamycin hereinafter referred to as (MNK agar plate) and incubated for 4 days at 27°C for the selection of transconjugants. The resulting colonies were purified by streaking on MNK agar plates as mentioned above. Thus, 1,400 transconjugants of *P. putida* [gene library of G. *oxydans* DSM No. 4025 in *P. putida*] were prepared.

### (2) Immunological screening of clones of the AADH gene of G. oxydans DSM No. 4025.

At first, 350 transconjugants (175 from *Sal*I library and 175 from *Eco*RI library) maintained MNK agar plates were individually cultivated in test tubes containing 5 ml of MNK medium. The cells were collected from 1.5 ml of each broth and treated for Western-blot analysis as follows. The cells were suspended in 50 µl of Laemmli buffer consisting of 62.5 mM Tris-HCl, pH 6.8, 10% glycerol, 5% mercaptoethanol and 2% SDS. The cell suspension was boiled for 3 minutes, and 10 µl of the cell lysate was applied on SDS-PAGE. The resulting protein bands were then electro-blotted to a nitrocellulose filter by an electroblotting apparatus (Marysol Industrial Co., Ltd.) operated at 40 V, 200 mA for 16 hours in 2.5 mM Tris-19.2 mM glycine buffer, pH 8.6, containing 20 % methanol. The filter was, then, incubated for 1 hour in 3% gelatin in TBS buffer consisting of 20 mM Tris, pH 7.5, and 500 mM NaCl. After a brief rinse in TTBS buffer consisting of 20 mM Tris, pH 7.5, 500 mM NaCl and 0.05% Tween 20, the filter was incubated for 1 hour with a first-antibody which contained 1 :500-diluted anti-AADH antibody in TTBS buffer containing 1% gelatin. The anti-AADH antibody had been prepared by mixing the AADH proteins purified from *G. oxydans* DSM No. 4025 with incomplete adjuvant, injecting the resulting mixture into a white rabbit twice with 2 weeks' interval, collecting whole blood 1 week after the second injection and preparing the serum fraction as the anti-AADH antibody. Then, the filter was washed twice (5 min each) in TTBS buffer and incubated for 1 hour in a second-antibody (goat anti-rabbit IgG-horseradish peroxidase conjugate) solution which contained 1:3,000-diluted second antibody in TTBS containing 1% gelatin. After washing in TTBS buffer twice and in TBS once, the filter was immersed in a color developing solution until blue bands became visible with Konica Immunostaining HRP Kit IS-50B (Konica, Tokyo, Japan) according to the supplier's recommendation. For an actual screening, five cell lysates were mixed and applied to one well for the first Western-blot screening. Out of 70 mixtures, 14 exhibited positive bands; nine samples had immuno-reactive proteins of approximate Mr 64,000, but two of these exhibited weak signals; one had an immuno-reactive protein of approximate Mr 60,000; and four samples had immuno-reactive proteins of Mr 55,000.

Seven mixture samples showing strong signals at Mr 64,000 were individually subjected to a second Western-blot screening to identify the clone in each mixture. One positive clone per one mixture samples was identified; plasmids of the seven clones were designated as p6E10, p16C8, p16F4, p17E8, p1E2, p24D4, and p26C3, respectively. By restriction enzyme analysis, it was found that four plasmids, p6E 10, p 16C8, p 16F4, and p17E8, carried the same DNA region and the other three carried different regions from that of the former four plasmids.

### (3) Screening of the AADH genes from the cosmid libraries by colony-blot and Southern-blot hybridization

To find the other AADH genes besides the genes obtained by the immunological screening as described above, the whole cosmid libraries of *G. oxydans* DSM No. 4025 in *E. coli* ED 8767 (*Sal*I-library and *Eco*RI-libraries) were screened by colony- and Southern-blot hybridization with a 0.9 kb *Sal*I fragment of p24D4. The 0.9 kb *Sal*I fragment hybridized with a oligonucleotide probe, ATGATGGT(GATC)AC(GATC)AA(TC)GT synthesized according to an internal amino acid sequence of the natural AADH enzyme purified from *G. oxydans* DSM No. 4025, MetMetValThrAsnValAspValGlnMetSerThrGlu. which was obtained by digestion and sequenced by automatic gas-phase sequencer (Applied Biosystems 470A). The cells of the cosmid libraries were appropriately diluted and spread on LK agar plates, and the resulting colonies were blotted onto nylon filters and were analyzed by hybridization with the ³²P-labeled 0.9 kb *Sal*I fragment. About 1% of the colonies showed positive signals; 41 colonies were selected from the *Sal*I library and 20 from *Eco*RI library, and they were subjected to restriction enzyme analysis, followed by Southern-blot analysis. Six different AADH gene-related DNA regions were isolated in this screening as follows: four already-isolated regions carried on p24D4, p1E2, p26C3 and, p17E8, and two new regions carried on two separate plasmids designated as pSS31 and pSS53. The other plasmid pSS33 carried both of the two regions which were carried on p24D4 and pSS31.

### (4) Immunological and enzymatic characterization of AADH clones

Western-blot analysis of cell lysates of *P. putida* carrying p24D4, p1E2, p26C3, pSS31 and p17E8 showed that the five clones encoded proteins with molecular weights of about 64,000, 62,500, 62,500, 60,000 and 62,000, respectively. Plasmid pSS33 encoded two immuno-reactive proteins with molecular weights of about 64,000 and 60,000, whereas pSS53 did not produce any immuno-reactive proteins.

Enzyme activities of each clone (cell free extract, soluble fraction and membrane fraction) were measured by photometric analysis. The cells of each clone were inoculated in 5 ml of MB medium in a test tube and cultivated at 30°C for 24 hours. The resulting broth was transferred into 200ml of fresh MB medium in 500 ml flask and the flask was shaken on the rotary flask shaker at 30°C for 24 hours. The cells were collected by centrifugation at 6,000 x g for 10 minutes and washed with 40 ml of cold buffer consisting of 50 mM Tris-HCl, pH 7.5, 5mM MgCl₂ and 0.5 mM phenylmethylsulfonyl fluoride and suspended with the same buffer to prepare cell suspension of 1 g wet cells per 5ml. The cell suspension was subjected twice to a French press cell disruptor (1,500 kg/cm²) and the resulting homogenate was centrifuged at 6,000 x g for 10 minutes to remove cell debris. Thus obtained cell free extract (CFE) was centrifuged at 100,000 x g for 60 minutes. The resulting supernatant and pellet were collected as the cytosol fraction and the membrane fraction, respectively and subjected to PMS-DCIP assay as follows. The enzyme reaction mixture (1.0 ml) contained 100 µM DCIP, 1mM PMS, 50 mM Tris malate-NaOH buffer, pH 8.0, a substrate and the enzyme (10 µl). Substrate-dependent decreasing rate of absorbance of DCIP at 600 nm was measured at 25°C by using a Kontron spectrophotometer UVIKON 810. Table 6 shows the level of enzyme activities in the cell free extract and the soluble fractions of the clones. According to the substrate specificity, the enzyme encoded on each plasmid was classified into large three groups, A-, B- and C-groups: A-group catalyzes the oxidation of L-sorbose, D-sorbitol and 1-propanol; B-group catalyzes the oxidation of D-glucose and D-sorbitol; C-group showed no clearly detectable activities on the substrates used. In the A-group, there were three types, A, A' and A" each of which was distinguished from each other by their physical map of the DNA carried on each plasmid. B- or C-group each consisted of only one type of protein derived from one region of the chromosomal DNA.

**Table 6.**

| Enzyme | Enzyme | Plasmid | CFE | | | Soluble | fraction | |
|---|---|---|---|---|---|---|---|---|
| Group | Name | | Sorbose 125 mM | Sorbose*1 125 mM | Glucose*2 50 mM | Sorbitol*3 125 mM | Sorbosone*4 2 mM | n-Propanol 50 mM |
| A | A | p24D4 | +++ | +++ | - | +++ | +++ | ++++ |
| A | A' | p1E2 | + | + | - | + | + | + |
| A | A" | p26C3 | + | + | - | +/- | + | + |
| B | B | pSS31 | - | - | ++++ | ++ | + | + |
| C | - | p17E8 | - | - | +/- | - | - | - |
| A and B | A and B | pSS33 | +++ | +++ | ++++ | ++++ | +++ | ++++ |

| | | | | | | | | |
|---|---|---|---|---|---|---|---|---|
| Level of the activity; ++++ :very high +++ : high +++ : medium + : low +/- : trace - : not detected *1 - *4:Oxidation product of each substrate was determined by a resting cell reaction followed by TLC analysis. * 1: Oxidation product of L-sorbose by Enzymes A, A', A", and [A and B] was 2KGA. *2: Oxidation product of D-glucose by Enzyme B, and Enzyems [A and B] was D-gluconic acid. *3: Oxidation product of D-sorbitol by Enzymes A, A', and A" was mainly D- glucose; that by Enzyme B was L-sorbose; and that by Enzymes [A and B] was mixture of D-glucose and L-sorbose. *4: Oxidation product of L-sorbosone by Enzymes A, A', A", B, and [A and B] was 2KGA. | | | | | | | | |

### Example 2. Nucleotide sequencing

Nucleotide sequences of the genes for Enzymes A, A', A" and B were determined with the plasmids, p24D4, p1E2, p26C3, and pSS31, respectively, by the dideoxynucleotide chain termination method using M13mp18 and M13mp19 (Boehringer Mannheim). One open reading frame (ORF) for each gene was found; the nucleotide sequences of the four genes are shown in the sequence list SEQ ID No. 1 to 4 and the amino acid sequences deduced from the nucleotide sequences were shown in the sequence list SEQ ID No. 5 to 8. The ORFs for Enzymes A, A', A" and B genes are 1737, 1737, 1734, and 1737-bp long and encode 579, 579, 578 and 579 amino acid residues all including 23 amino acid of signal sequences.
The homologies between Enzymes A, A', A" and B are shown in Table 7.

**Table 7. Homologies of amino acid sequences among AADHs.**

| | | | | |
|---|---|---|---|---|
| (%) | | | | |
| | Enzyme A | Enzyme A' | Enzyme A" | Enzyme B |
| Enzyme A | 100 | - | - | - |
| Enzyme A' | 89 | 100 | - | - |
| Enzyme A" | 85 | 86 | 100 | - |
| Enzyme B | 83 | 82 | 81 | 100 |

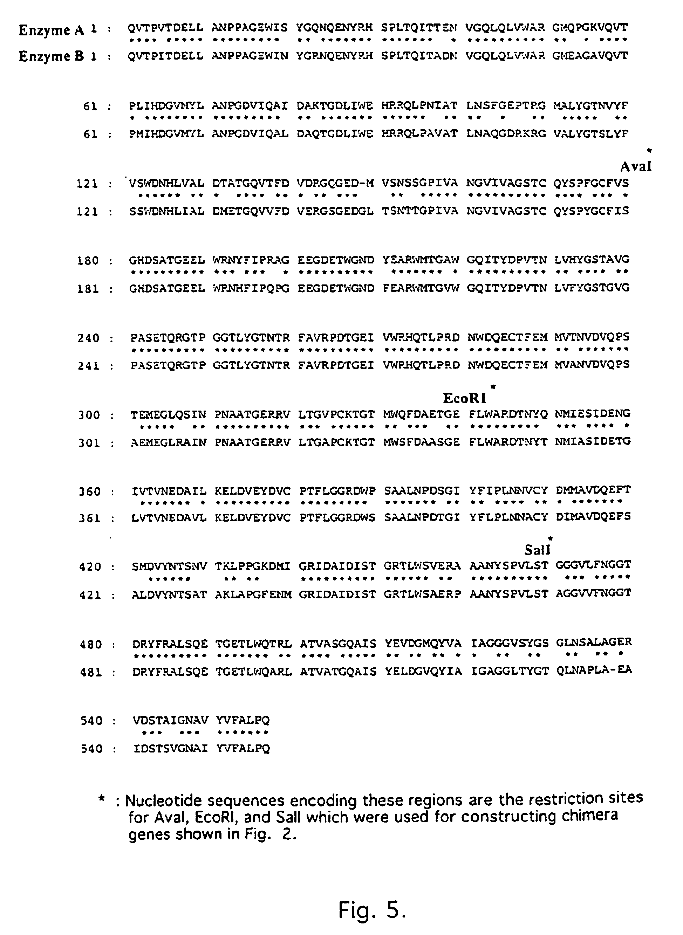

Figure 5 shows the amino acid sequences of mature Enzyme A and Enzyme B which are aligned so as to be comparable.

Homology search of Enzymes A, A', A" and B revealed that Enzymes A, A', A" and B showed rather low homology (26-31% homology through the polypeptides) with several quino-proteins including alcohol dehydrogenase of *Acetobacter aceti* (T. Inoue et al., J. Bacteriol. 171: 3115-3122) or *Acetobacter polyoxogenes* (T. Tamaki et al., B. B. A., 1088:292-300), and methanol dehydrogenase of *Paracoccus denitrificans* (N. Harms et al., J. Bacteriol., 169: 3966-3975), *Methylobacterium organophilum* (S. M. Machlin et al., J. Bacteriol., 170: 4739-4747), or *Methylobacterium extorquens* (D.J. Anderson et al., Gene 90: 171-176).

### Example 3. Subcloning of AADH genes

Enzyme A gene was originally cloned as a cosmid clone of p24D4 which has about 25 kb insert in *Eco*RI site of pVK100. Then, it was further subcloned to use as an Enzyme A gene cassette. The 2.7 kb EcoRV fragment which includes ORF of Enzyme A gene with about 500 bp of non-coding regions at the both ends was excised from 3.4 kb *Nru*I fragment, which was isolated from p24D4 in M 13 mp18, and was ligated to *Hind*III site of pUC18 with *Hind*III linker (CAAGCTTG). The resulting plasmid was designated pSSA202. Enzyme A gene cassette (2.7kb *Hind*III fragment) was then inserted at *Hind*III site of pVK102 to produce pSSA102R. The plasmid pSSA102R was introduced into nalidixic acid resistant *P. putida* [ATCC 21812] by a conjugal mating method as described in Example 1-(1). The transconjugant of *P. putida* carrying pSSA102R was selected on MB agar medium containing 50 µg/ml nalidixic acid and 10 µg/ml tetracycline (MNT agar medium) and subjected to a mini-resting cell reaction. The reaction mixture (100 µl) consisting of 20 g/l L-sorbose, 3 g/l NaCl, 10 g/l CaCO₃ and the cells collected from the MNT agar culture with a toothpick was incubated at room temperature with gentle shaking for 24 hours. The reaction mixture was assayed with TLC and 2KGA was identified as the product, while no 2KGA was observed by the same resting cell reaction with the host, nalidixic acid resistant *P. putida* [ATCC 21812].

Enzyme B gene was originally cloned as a cosmid clone of pSS31 which has about 30kb insert in *Sal*I site of pVK102. It was subcloned as 6.5kb *Bgl*II fragment into *Bgl*II site of pVK101 (ATCC 37157) to obtain pSSB102. Then, it was further subcloned to use as a Enzyme B gene cassette. The 6.5 kb *Bgl*II fragment was cloned into *Bam*HI site of pUC 18 to obtain pSSB202. Then, 2.3 kb *Xho*II fragment was excised from pSSB202. The 2.3 kb *Xho*II fragment includes ORF of Enzyme B with 120 bp of 5'-noncoding region and about 500 bp of 3'-noncoding region. The fragment was treated with Klenow fragment to fill-in the cohesive ends and cloned into *Hind*III site of pUC 18 with *Hind*III linker to produce pSSB203. The Enzyme B gene cassette (2.3 kb *Hind*III fragment) was inserted at *Hind*III site of pVK102 to make pSSB 103R. The plasmid pSSB 103R was introduced into nalidixic acid resistant *P. putida* [ATCC 21812] by a conjugal mating method, and the transconjugant of *P. putida* carrying pSSB 103R was selected on MNT agar medium and subjected to a mini-resting cell reaction. *P. putida* carrying pSSB 103R showed the Enzyme B actiuvity (L-sorbose formation from D-sorbitol) in the resting cell reaction. (Incidentally, XhoII fragment was found not to be a *Xho*II*-Xho*II fragment, but a *Xho*II-*Xho*I fragment as a result of nucleotide sequencing. *Xho*I might be present in the *Xho*II preparation.)

Enzyme A' and Enzyme A" genes were originally cloned as a cosmid clone of p1E2 and p26C3 which have about 30 kb insert in *Sal*I site of pVK102 and further subcloned basically as described above. Enzyme A' gene in 3.5 kb *Xho*II fragment was subcloned in *Bgl*II site of pVK102 to construct pSSA'101R, and Enzyme A" gene in 2.7 kb *Eco*RV fragment was first subcloned into M13mp19 and then re-subcloned between *Hind*III and *Bgl*II sites of pVK102 to construct pSSA"102.

### Example 4. Isolation and characterization of AADHs from transconjugants of P. putida.

### (1) Cultivation of microorganisms

*P. putida* [ATCC 21812] carrying cosmid vector pVK102 containing the Enzyme A, A', A" and B genes; pSSA 102R, p1E2, p26C3 and pSSB103R, respectively, were cultivated in MB broth in the presence of antibiotic. Antibiotics added into medium were as follows; 5 µg/ml tetracycline for pSSA 102R (Enzyme A) and pSSB 103R (Enzyme B), 25 µg/ml kanamycin for p1E2 (Enzyme A') and p26C3 (Enzyme A"). From the agar plate of MB containing the respective antibiotic, the cells were inoculated in 10 test tubes containing 5 ml MB medium with the respective antibiotic and cultivated with shaking at 30°C. After 2 days of cultivation, the cells were transferred to ten 500 ml-Erlenmeyer flasks containing 100 ml of the same medium and cultivated with shaking at 30°C. After 1 day of cultivation, the seed cultures were combined and transferred to 18 liters of the medium in 30 L jar fermenter (Marubishi) and cultivated for 18 hours with 300 rpm agitation and 1.0 vvm aeration at 30°C. The cells were harvested by centrifuge at 6,000 x g for 10 minutes, washed once with 1.5 liters of 25 mM Tris-HCl, pH 7.5, containing 5 mM CaCl₂, 1 mM MgCl₂, 0.2 M NaCl. 2.5% sucrose, and 0.5 mM PMSF and stocked at -20°C until use. As a result, about 150 g wet weight cells were obtained.

### (2) Purification of the cloned Enzymes A, A', A", and B.

Purifications of the Enzymes A, A', A" and B were carried out by the same procedure with almost the same scale. All operations were carried out at 4 - 10°C unless otherwise stated. The enzyme activity determination for Enzyme A, A', A" and B were carried out with the substrates, L-sorbose, n-propanol, n-propanol and D-glucose, respectively, by spectrophotometric assay as described in Example 1 throughout the purification steps. The cells (about 100 g wet weight cells containing 8 - 10 g of total proteins) were thawed and suspended in about 200 ml of 25 mM Tris-HCl, pH 8.0, and disrupted by passing through French press (1500 kg/cm²) twice. Then, DNase and MgCl₂ were added to the suspension at the final concentration of 0.01 mg/ml and 1 mM, respectively, to reduce viscosity of the solution due to DNA. Cell debris was removed by centrifugation at 6,000 x g for 10 minutes. The suspension was filled up to 240 ml with the 25 mM Tris-HCl buffer, pH 8.0, and centrifuged at 100,000 x g for 90 minutes to remove insoluble membrane fraction. The soluble supernatant was filled up to 240 ml with the Tris buffer and, then, pyrroloquinoline quinone (PQQ) and CaCl₂ were added at the final concentration of 12.5 µM and 5 mM, respectively, and the solution was stirred vigorously for 15 minutes at room temperature. The soluble fraction prepared as above was fractionated by (NH₄)₂SO₄. The fraction 35 - 60%-saturated (NH₄)₂SO₄ was precipitated and resuspended in 100 ml of 25 mM Tris-HCl buffer, pH 8.0, containing 5 mM CaCl₂, and 5% sucrose and, then, PQQ was added again at the final concentration of 12.5 µM. The enzyme solution was dialyzed against 1000 ml of the same buffer (without PQQ) overnight. Twenty grams of solid polyethylene glycol #6000 was added to the dialysate slowly with gentle stirring. After stirring for 30 minutes, precipitates were removed by centrifugation at 10,000 x g for 20 minutes, and the supernatant was filled up to 200 ml with the buffer indicated as above.

The enzyme solution prepared as above was purified by following three chromatography steps.
The first step: DEAE-Toyopearl 650M

The crude enzyme solution was subjected to a column of DEAE-Toyopearl 650M (2.5x 40 cm) which had been equilibrated with 25 mM Tris-HCl buffer, pH 8.0, containing 5 mM CaCl₂, and 5% sucrose. The column was washed with 400 ml of the same buffer and the enzyme was eluted by 2,000 ml of 0 - 0.5 M NaCl linear gradient in the buffer at a flow rate of 150 ml/hour. The enzyme active fractions were pooled and diluted 2-fold with the buffer without NaCl.
The second step: Q-Sepharose (Fast Flow)

The enzyme solution was subjected to a column of Q-Sepharose (Fast Flow) (1.5 x 20 cm) which had been equilibrated with the buffer without NaCl. The column was washed with 200 ml of the buffer containing 0.2 M NaCl and the enzyme was eluted by 600 ml of 0.2 - 0.6 M NaCl linear gradient in the buffer at a flow rate of 50 ml/hour. The enzyme active fractions were pooled and concentrated to 2.5 ml by using ultrafilter:Amicon, PM-30 under N₂ gas.
The third step: Sephacryl S-300 HR (gel filtration)

The concentrated enzyme was filtrated by a column of Sephacryl S-300 HR (2.5 x 100 cm) which had been equilibrated with 25 mM HEPES, pH 7.5, containing 5 mM CaCl₂, 5% sucrose, and 0.2 M NaCl. The column was developed by the same buffer at a flow rate of 20 ml/hour. The enzyme active fractions were pooled and concentrated to below 1 ml by the ultrafilter mentioned above and, then, stocked at -80°C. The enzymes concentrated in the HEPES buffer was stable for at least 2 months at -80°C.

Consequently, 26.0 mg of Enzyme A, 0.35 mg of Enzyme A', 0.41 mg of Enzyme A", and 5.0 mg of Enzyme B were obtained.

### (3) Properties of the Enzymes A, A', A" and B.

### a) Molecular weight and subunit.

The Enzymes A, A', A" and B were eluted at the same position from the same gel filtration column on Sephacryl S-300HR under the same condition. The molecular weight of the enzymes was estimated as approximately 135,000 comparing with the molecular weight standard proteins (SDS-PAGE Standards, Low Range, Bio-Rad Laboratories, Richmond, CA, USA). The Enzymes A, A', A" and B showed homogeneous single bands on SDS-PAGE analysis with molecular weights of 64,000, 62,500, 62,500 and 60,000, respectively. All the Enzyme bands A, A', A" and B were detected on Western blotting analysis using anti-AADH rabbit serum. Therefore, it was concluded that the enzymes consisted of two identical subunits as an homo-dimeric form.

### b) N-terminal amino acid sequence and amino acid composition.

N-terminal amino acid sequences of the mature Enzymes A, A" and B were analyzed with automatic gas-phase sequencer (470A; Applied Biosystems) by Edman method [Acta Chem. Scand., 4, 283-293, (1950)]. The analysis of the Enzyme A' was not done because of an insufficient purity of the sample. The results were as follows:
Enzyme A : Gln-Val-Thr-Pro-Val-Thr---
Enzyme A" : Blocked N-terminal residue
Enzyme B : Gln-Val-Thr-Pro-Ile-Thr-Asp-Glu-Leu-Leu-Ala----.
The determined sequences of Enzyme A and B were identical to the sequences (starting from the twenty-fourth residues) deduced from the nucleotide sequences described in SEQ ID No. 5 and 8; these results indicate that the initial 23 residues of the enzymes are the signal sequences. By analogy of the Enzymes A and B, the first 23 residues of Enzyme A' and A" are also deduced to be the signal sequences.

The amino acid composition of the Enzyme A was determined. The protein was hydrolyzed with 6 N HCl at 110°C for 24 hours or 4 M methanesulfonic acid (after oxidation with performic acid) at 115°C for 24 hours. Amino acid analysis was performed by using Kontron amino acid analyzer (ninhydrin system). The analytical data were compared with the amino acid composition deduced from the DNA sequence of Enzyme A gene. It indicated that the purified Enzyme A was certainly a product of the Enzyme A gene.

### c) Substrate specificity

The Enzymes A, A', A" and B were characterized by their substrate specificities on PMS-DCIP assay as described above using 8 substrates, n-propanol, isopropanol, D-glucose, D-sorbitol, L-sorbosone, D-mannitol, L-sorbose, and D-fructose. The results were indicated in Table 1.

### d) Physicochemical property

Physicochemical studies of optimal pH, pH stability and thermal-stability, of the Enzymes A (as L-sorbose dehydrogenase activity), A' (as n-propanol dehydrogenase activity), A" (as L-sorbose dehydrogenase activity) and B (as D-sorbitol dehydrogenase activity), were performed by the PMS-DCIP assay.

Table 2 summarizes the results of optimal pH of the enzymes. The enzyme activity was assayed by the PMS-DCIP spectrophotometric assay using various pH buffers. The buffers were 50 mM Tris-malate-NaOH, pH 6.0, 6.5, 7.0, 7.5, 8.0 and 8.5; 50 mM glycine-NaOH, pH 9.0 and 9.5. The extinction coefficients of DCIP at pH 6.0, 6.5, 7.0, 7.5, 8.0, 8.5, 9.0 and 9.5 were taken as 10.8, 13.2, 14.5, 14.9, 15.0, 15.1, 15.1 and 15.1, respectively. All the enzymes showed their optimal points at pH 8.0 - 8.5. The Enzymes A" and B had relatively wide pH range toward lower pH, compared with the Enzymes A and A'.

Table 3 indicates the results of pH-stabilities of the enzymes. The enzyme (about 0.01 mg/ml) was incubated with 50 mM buffer containing 5% sucrose, 0.2 M NaCl, and 5mM CaCl₂ at 25°C for 3 hours and assayed by PMS-DCIP spectrophotometric method. The buffers were Na-acetate, pH4 and 5, Tris-malate-NaOH, pH 6, 7 and 8, glycine-NaOH, pH 9 and 10. The values in the table are expressed as relative activity against that obtained by no incubation at pH 8.0. The substrates used for the enzymes were 125mM L-sorbose for Enzymes A and A", 50 mM n-propanol for Enzyme A', and 125 mM D-sorbitol for Enzyme B. Profiles of pH-stabilities of Enzymes A, A', A", and B were almost the same; they were stable at the range of pH 6 to 9.

Table 4 indicates the results of thermal-stabilities of the enzymes. The enzyme (about 0.05 mg/ml) in 25 mM HEPES buffer, pH 7.5, containing 5% sucrose, 0.2M NaCl, and 5 mM CaCl₂ was incubated at temperature indicated in the table (4 - 60°C) for 5 minutes, cooled in ice bath and assayed by PMS-DCIP spectrophotometric method. Remaining activity was expressed as relative activity against that obtained by 4°C incubation. The substrates used for the enzymes were 125mM L-sorbose for Enzyme A and Enzyme A", 50 mM n-propanol for Enzyme A', and 125 mM D-sorbitol for Enzyme B. After the treatment of the enzymes at 40°C for 5 min, the residual activity of Enzyme A was 20%, and those of Enzymes A', A", and B were 70 - 85%.

### e) General inhibitors

The enzyme (about 0.05 mg/ml) in 25 mM HEPES buffer, pH 7.5, containing 5% sucrose was incubated with metal or inhibitor for 30 minutes at 25°C. Remaining activity was assayed by PMS-DCIP spectrophotometric assay as described in Example 1. Remaining activity is expressed as relative activity against blank incubation. Effects of metal ions on the enzymes are listed in Table 5. MgCl₂ and CaCl₂ were nearly inert to the enzymes, while the other metal ions, especially CuCl₂, significantly affected. Effects of inhibitors on the enzymes are also included in Table 5. EGTA and EDTA inhibited the Enzyme A, A' and A", remarkably. However, Enzyme B was less inhibited than the A group enzymes by EDTA and EGTA.

### Example 5. Efficient production of Enzyme B in E. coli

The signal peptide region of the Enzyme B was replaced with that of maltose binding protein (malE) of *E. coli* as follows. Two oligonucleotides (SEQ ID No. 9 and 10) were synthesized with Applied Biosystem 381A DNA synthesizer and annealed to generate a double-stranded DNA fragment encoding a amino acid sequence (SEQ ID No. 11), MetLysIleLysThrGlyAlaArgIleLeuAlaLeuSerAlaLeuThrThrMetMetPheSer AlaSerAlaLeuAla(Gln), which was, then, treated with T4 polynucleotide kinase [J. Biol. Chem., 259, 10606-10613, (1984)]. pSSB203 (see Example 3) was digested with the restriction enzyme *Sph*I*,* treated with T4 DNA polymerase and digested with *Bst*P1. The resulting 1.72 kb DNA fragment carrying Enzyme B gene without the region coding for the original signal sequence and the first amino acid residue (Gln) of the mature Enzyme B was isolated from an agarose gel after agarose gel electrophoresis. The *E. coli* expression vector, pTrc99A (Pharmacia Co., Uppsala, Sweden), which was digested with the restriction enzymes *Nco*I (at ATG start codon) and *Sma*I was ligated with above two DNA fragments. The resulting plasmid was designated as pTrcMal-EnzB and used to transform *E. coli* JM109. The transformant was grown in two 2-liter flasks each containing 600 ml of LB with 100 µg/ml ampicillin at 28°C and IPTG was added to 0.1 mM when cell concentration reached at about 1.5 OD600. Following the addition of IPTG, the cells were cultivated for an additional 3 - 4 hours. The cells were harvested by centrifugation (4,000 x g) at 25°C for 10 minutes, suspended with 500 ml of 30 mM Tris-HCl, pH 8.0, containing 20% sucrose at 25°C. After EDTA was added to 1 mM into the cell suspension, the cells were incubated with gentle shaking for 5 minutes at 25°C and collected by centrifugation (8,000 x g) at 4°C for 15 minutes. The cells were resuspended with 500 ml of ice cold 5 mM MgSO₄ solution and incubated with gentle shaking for 5 minutes at 4°C. The cell suspension was centrifuged at 8,000 x g for 10 minutes at 4°C to obtain a supernatant as a cold osmotic shock extract, which was found to contain the Enzyme B protein (a molecular weight of 60,000) with the purity more than 50 - 60% by SDS-PAGE analysis. The supernatant was first supplemented with Tris-HCl, pH 8.0, to 20 mM, and incubated at 25°C firstly with EDTA at 10 mM final concentration for 10 min, secondly with CaCl₂ at 20 mM final concentration for 10 minutes and lastly with PQQ at 25 µM final concentration. For stabilization of the enzyme, α-methyl-D-glucoside (a competitive inhibitor) was added to 20 mM final concentration in the supernatant. The Enzyme B was completely purified by following two chromatographies. At first, the supernatant was loaded onto a Q-Sepharose column (1.6 x 12 cm) which had been equilibrated with 20 mM Tris-HCl, pH 8.0, containing 1 mM CaCl₂ and 20 mM α-methyl-D-glucoside, and the Enzyme B was eluted with 600 ml of 0 - 0.4 M NaCl linear gradient in the same buffer. A red protein peak eluted at about 0.25 M NaCl was collected and concentrated to about 0.5 ml by Centricon-30 (Amicon). Finally, the Enzyme B was passed through a SephacrylS-300HR column with 20 mM HEPES, pH 7.8, containing 0.2 M NaCl, 1 mM CaCl₂ and 20 mM α-methyl-D-glucoside. A red protein peak eluted around a molecular weight of 135,000 daltons position was collected as the final purified Enzyme B. Consequently, about 8 mg of the purified Enzyme B was obtained from 1.2 liters cultivation broth of *E. coli .*

### Example 6. Host-vector system

A host-vector system for *G. oxydans* [DSM No. 4025] was established by using the conjugal mating system with a broad-host-range cosmid, pVK102. Initially, only one transconjugant was isolated from *G. oxydans* [DSM No. 4025] having nalidixic acid resistance. A new host, GOS2, was isolated from the transconjugants, *G. oxydans*[DSM No. 4025] carrying pVK102 by curing pVK102. A second host, GOS2R, was then derived from the GOS2 by adding rifampicin resistance (100 µg/ml), which enables easy selection of the transconjugants from the donor *E*. *coli.* The plasmid transfer frequency into GOS2R was 10⁻³ ~ 10⁻⁴ transconjugants/recipient. The 2KGA productivity of GOS2R, however, was about 10% lower than that of *G. oxydans* [DSM No. 4025]. The third host, GORS6-35, was obtained from *G. oxydans* [DSM No. 4025] by selecting the strain with rifampicin resistance, high 2KGA productivity and relatively high competence through a series of experiments, including the conjugation, curing and 2KGA fermentation.

### (1) Isolation of GOS2

Resistance to nalidixic acid was added to *G. oxydans* [DSM No. 4025]. Cells of *G. oxydans* [DSM No. 4025] were streaked onto Trypticase Soy Broth (BBL, Becton Dickinson Microbiology Systems Cockeysville, MD. USA) (T) agar medium with 50 µg/ml of nalidixic acid (TN agar medium) and incubated at 27°C for 5 days. The resulting colonies were again streaked on the same agar plates to obtain a nalidixic acid-resistant *G. oxydans* DSM No. 4025, GON. The broad-host-range cosmid pVK102 (Km^{r}, Tc^{r}) was transferred from *E. coli* carrying pVK102 into the GON strain by the tri-parental conjugal mating as follows. A helper strain, *E*. *coli* carrying pRK2013 and a donor strain carrying pVK102 were cultivated in LB medium with 50 µg/ml of kanamycin at 37°C overnight. The cultures were transferred to fresh LB medium with kanamycin and incubated for 5-6 hours. Recipient strain, GON, was cultivated in TN liquid medium at 30°C overnight. *E*. *coli* and GON strains were separately centrifuged and re-suspended in equal- and one tenth- volumn of fresh T medium, respectively. One hundred µl of each cell suspension was mixed together and 30 µl portion of the mixture was spotted onto a nitrocellulose filter placed on the surface of a NS2 agar plate. Transconjugants were selected on the T agar medium containing 50 µg/ml of nalidixic acid and 50 µg/ml of kanamycin (TNK agar medium). Several colonies were obtained on the selection plates where many spontaneous mutants of *E. coli* (Nal^{r}, Km^{r}) colonies also appeared. The plasmid and chromosomal DNAs of the transconjugant candidates were prepared and compared with the authentic pVK102 and chromosomal DNA of *G. oxydans* DSM No. 4025 by restriction analysis and Southern-blot hybridization. Consequently, one transconjugant of *G. oxydans* [DSM No. 4025] carrying pVK102, GON8- 1, was identified. The plasmid DNA prepared from GON8-1 was identical to that of pVK102 and replicable in *E. coli.* The chromosomal DNA of GON8-1 was identical to that of *G. oxydans* [DSM No. 4025].

To isolate strains that could work as hosts with higher competence for conjugal mating, the transconjugant GON8-1 was cured of the plasmid pVK102. GON8-1 was cultivated in T broth without antibiotics at 30°C for 2 days, 2% of the culture was transferred into fresh T broth. After three such cultivation cycles, the cells were spread on T agar plates, incubated at 27°C for 4 days, and the resulting colonies were picked onto TNK and TN agar plates to select Km^{s} strains. One of the Km^{s} strains was designated as GOS2 and was confirmed by Southern-blot hybridization not to be carrying any DNA region of pVK102. Then, pVK102 was transferred into strain GOS2 by a conjugal mating; this strain showed 10² ~ 10³ fold higher competence (namely 10⁻⁵ ~ 10⁻⁶ transconjugants / recipient) than *G. oxydans* [DSM No. 4025] did.

### (2) Isolation of GOS2R, a rifampicin-resistant mutant of GOS2.

Rifampicin resistant (Rif^{T}) mutants from GOS2 were isolated through repeated transfer of GOS2 cells onto T agar medium containing 20 ∼ 100 µg/ml rifampicin; one of the Rif^{T} strains was designated as GOS2R. Strain GOS2R showed very high competence; 10⁻² ∼ 10⁻³ and 10⁻⁴transconjugants /recipient on TRK agar (T agar medium containing 100 µg/ml rifampicin and 50 µg/ml kanamycin) plate and on TRT agar (T agar medium containing 100 µg/ml rifampicin and 3 µg/ml tetracycline) plate, respectively.

2KGA productivity from L-sorbose by GOS2R was compared with that of *G. oxydans* [DSM No. 4025]. The cells maintained on NS2 agar medium were inoculated into 5 ml of the seed culture medium consisting of 8% L-sorbose (sterilized separtely), 0.05% glycerol, 0.25% MgSO₄·7H₂O, 1.75% corn steep liquor, 5.0% baker's yeast, 1.5% CaCO₃, and 0.5% urea (sterilized separately) (pH 7.0 before sterilization) and incubated at 30°C for 24 hours. The resulting seed culture (5 ml) was inoculated into a 500-ml Erlenmeyer flask containing 50 ml of the production medium PMS 10 consisting of 10% L-sorbose, (sterilized separtely), 0.05% glycerol, 0.25% MgSO₄·7H₂O, 3% corn steep liquor, 6.25% baker's yeast, 1.5% CaCO₃, and 1.6% urea (sterilized separately) (pH 7.5 before sterilization) and incubated at 30°C for 4 days with shaking (180 rpm). The quantitative determination of 2KGA was assayed by high performance liquid chromatography. GOS2R and *G. oxydans* [DSM No. 4025] produced 87.3 and 97.3 g/l of 2KGA, respectively.

### (3) Isolation of GORS6-35 as a host with high 2KGA productivity

To evaluate the self-cloning of AADH genes in the strain with the same productivity of 2KGA from L-sorbose as *G. oxydans* [DSM No. 4025], a new host was constructed by (i) adding rifampicin-resistance (200 µg/ml), (ii) introducing and curing pVK102, and (iii) selecting 2KGA high producer from L-sorbose. Thus obtained GORS6-35 shows the following two characteristics: (i) almost the same 2KGA productivity (about 100 g/l 2KGA from 10% L-sorbose) as the parent *G. oxydans* [DSM No. 4025]; and (ii) a competence (10-6 ∼ 10⁻⁷ transconjugants /recipient).

### Example 7. Construction of promoter-replaced Enzyme B gene.

The promoter of Enzyme A gene (PA) is likely strong in *G. oxydans* [DSM No. 4025] because Enzyme A was found to be one of the highest-expressing proteins in amount in the cell when total cell free extract of *G*. *oxydans* [DSM No. 4025] was subjected to SDS-polyacrylamide gel electrophoresis and the resulting gel was stained with Coomassie Brilliant Blue R-250. The PA and another promoter, a promoter of kanamycin resistant gene of Tn5 (PTn5), which could express the kanamycin resistance in *G. oxydans* [DSM No. 4025], were attached to the structure gene with the SD sequence of Enzyme B gene as shown in Fig. 10.

Enzyme B gene-containing 2.3 kb *Hind*III fragment was inserted in M 13 mp 18 and the resulting phage DNA was subjected to site-directed mutagenesis carried out with T7-GEN^{™} In Vitro Mutagenesis Kit (TOYOBO Co., Ltd., Osaka Japan) according to the recommendations by the supplier (Fig. 9). To insert various promoters upstream of Enzyme B gene instead of Enzyme B promoter, *Bam*HI site was created upstream of the SD sequence. A primer for the mutagenesis, GTTAGCGCGGTGGATCCCCATTGGAGG (27-mer including *Bam*HI site, SEQ-IDNo. 12), were synthesized with Applied Biosystems 381A DNA synthesizer. The resulting *Bam*HI*-Hind*III fragment carries Enzyme B SD and structural genes without the Enzyme B promoter (PB).

Then promoter of Enzyme A gene (PA) was subcloned by PCR method using primers tagged with the sequences for the *Hind*III and *Bam*HI sites. The PCR reaction was carried out with GeneAmpTM DNA Amplification Reagent Kit (Takara Shuzo, Kyoto, Japan) with a thermal cycler, Zymoreactor II (Atto Corp., Tokyo, Japan). The reaction consists of pre-treatment before adding enzyme (94°C, 5 minutes.); 30 cycles of denaturation step (94°C, 1 minute.), annealing step (60°C, 1 minute.), and synthesis step (72°C, 1minute.); and post-treatment (72°C, 5 minutes.). Plasmid pSSA202 (pUC18-Enzyme A gene in 2.7kb *Hind*III) was used as the template DNA. The reaction mixture contained 200 µM of dNTPs, 1 µM of each primer, 1 ng of template DNA and 2.5 u of AmpliTaqTM DNA polymerase in the buffer supplied. Consequently, 300 bp fragment upstream from the SD sequence was amplified. The PCR product was inserted into pUC18 between *Hind*III and *Bam*HI sites and used for nucleotide sequencing; the amplified sequences do not have any mutations caused by misincorporation in PCR.

The promoter of the kanamycin resistant gene, PTn5, was first obtained as a *Hind*III-*Pst*I fragment from the plasmid pNeo (Pharmacia Co., Uppsala, Sweden). The *Hind*III*-Pst*I fragment was then inserted into the multicloning site of pUC18, and finally the PTn5 was excised as a *Hind*III*-Bam*HI fragment.

The *Hind*III*-Bam*HI fragments containing the PA and PTn5 promoters were inserted in the *Hind*III site of pUC18 together with *Bam*HI*-Hind*III fragment containing the PB promoter-removed Enzyme B structural gene. The *Hind*III fragments from the resulting plasmids were subcloned into pVK100 to produce pSSAP-B and pSSPTn5-B, which were transferred into GOS2R by conjugal mating as described in Example 6.

### Example 8. 2KGA production by transconjugants of GOS2R in flask

### (1) 2KGA production from L-sorbose by Enzyme-A gene-amplified transconjugant in single culture fermentation in flask.

The Enzyme A plasmid, pSSA102R, and the vector plasmid, pVK102, were introduced into GOS2R by a conjugal mating method as described in Example 6. The resulting transconjugants were maintained on NS2 agar medium containing 30 µg/ml tetracycline and subjected to 2KGA fermentation from L-sorbose. The cells of the transconjugants were inoculated into 5 ml of the seed culture medium described in Example 6 and incubated at 30°C for 24 hours. The resulting seed culture (5 ml) was inoculated into a 500-ml Erlenmeyer flask containing 50 ml of the PMS 10 production medium described in Example 6 or the PMS12 production medium consisting of 12% L-sorbose, (sterilized separtely), 0.05% glycerol, 0.25% MgSO₄·7H₂O, 3% corn steep liquor, 10 % baker's yeast, 1.5% CaCO₃, and 2% urea (sterilized separately) (pH 7.5 before sterilization) and incubated at 30°C for 4 or 5days with shaking (180 rpm). As a result, GOS2R (pSSA102R) and GOS2R (pVK102) produced 92.2 and 89.1 g/l 2KGA, respectively, from 10% L-sorbose in 4 days, and 105.7 and 99.9 g/l 2KGA, respectively, from 12% L-sorbose in 5 days.

### (2) 2KGA production from D-sorbitol by GOS2R (pSSB 103R) in single culture fermentation in flask.

The Enzyme B plasmid, pSSB 103R, and the vector plasmid, pVK102, were introduced into GOS2R by a conjugal mating method as described in Example 6. The resulting transconjugants were maintained on NS2 agar medium containing 30 µg/ml tetracycline and subjected to 2KGA fermentation from D-sorbitol. The cells of the transconjugants were inoculated into 5 ml of the seed culture medium consisting of 8% D-sorbitol, 0.05% glycerol, 0.25% MgSO₄·7H₂O, 1.75% com steep liquor, 5.0% baker's yeast, 1.5% CaCO₃, and 0.5% urea (sterilized separately) (pH 7.0 before sterilization) and incubated at 30°C for 24 hours. The resulting seed culture (5 ml) was inoculated into a 500-ml Erlenmeyer flask containing 50 ml of three production media shown in Table 8 and incubated at 30°C for 3 days with shaking (180 rpm). As a result, GOS2R (pSSB 103R) produced about 61.5, 71.5 and 73.0 g/l of 2KGA from 8%, 10% and 12% D-sorbitol, respectively, while GOS2R (pVK102) produced 19.5,25.4 and 30.2 g/l 2KGA, respectively.

**Table 8.**

| | | | (%) |
|---|---|---|---|
| Ingredients | PMSL8 | PMSL10 | PMSL 12 |
| D-Sorbitol | 8.0 | 10.0 | 12.0 |
| Glycerol | 0.05 | 0.05 | 0.05 |
| MgSO₄·7H₂O | 0.25 | 0.25 | 0.25 |
| CSL | 3.0 | 3.0 | 3.0 |
| Baker's yeast | 5.0 | 6.25 | 10 |
| Urea* | 1.25 | 1.6 | 2.0 |
| CaCO₃ | 1. 5 | 1. 5 | 1.5 |

| | | | |
|---|---|---|---|
| pH 7.5 before sterilization *: sterilized separately | | | |

### (3) 2KGA production from D-sorbitol by GOS2R (pSSAP-B) and GOS2R (pSSPTn5-B) in single culture fermentation in flask.

The cells of GOS2R (pSSAP-B), GOS2R (pSSPTn5-B) and GOS2R (pSSB 103R), GOS2R (pVK100) were cultivated in the PMSL10 production medium in Erlenmeyer flasks at 30°C for 3 days as described in Example 8 (2). The amounts of 2KGA produced were shown in Table 9.

**Table 9.**

| The amount of 2KGA (g/l) | | | |
|---|---|---|---|
| Strain | 1 day | 2 days | 3 days |
| GOS2R (pSSAP-B) | 47.2 | 67.0 | 67.7 |
| GOS2R (pSSPTn5-B) | 23.4 | 28.6 | 29.4 |
| GOS2R (pSSB103R) | 30.5 | 54.3 | 62.7 |
| GOS2R (pVK100) | 10.2 | 18.3 | 19.3 |
| GOS2R | 6.7 | 14.7 | 16.4 |

### Example 9 2KGA production from D-sorbitol in 3-L Jar fermentations by single microorganism

### (1) Single culture fermentation by GOS2R (pSSB 103R)

Five ml portions of the seed culture prepared in test tubes as described in Example 8-(2) were transferred to four 500-ml Erlenmeyer flasks containing 50 ml of the same seed culture medium and incubated at 30°C for 24 hours with shaking (180 rpm). The resulting broth (200 ml of the seed culture) was inoculated into 3-L jar fermentor containing 1800 ml of the PMSL10 production medium containing 3ml of antifoam. The fermentor was operated at 30°C, 700 rpm and 0.5vvm. D-Sorbitol was fed in ways: (i) 200 ml of 50% D-sorbitol was fed in 6 hours from the 24^{th} to the 30^{th} hour; or (ii) 280 ml of 50% D-sorbitol was fed in 8.4 hours from the 24^{th} to the 32.3^{th} hour. As a result, 99.0 and 103.4 g/l 2KGA were produced by the fed-batch fermentations (i) and (ii), respectively in 51 hours.

### Example 10 2KGA production from D-sorbitot by Enzyme B gene-amplified GOS2R in mixed culture fermentation with E. coli in flask

### (1) Mixed-culture fermentations with B. megaterium, E. coli and P. putida.

*B. megaterium* [DSM No. 4026]. E. *coli* HB 101 and *P. putida* [ATCC 21812], growth factor suppliers, were cultivated in 150 ml of the seed culture medium consisting of 0.3% yeast extract (Difco), 0.3% beef extract (Kyokuto Seiyaku, Tokyo, Japan), 3% corn steep liquor, 1% polypeptone (Kyokuto), 0.1% urea, 0.1 % KH₂PO₄, 0.02% MgSO₄·7H₂O, 2% L-sorbose, 0.1% CaCO₃ (pH 7.1 before sterilization) for 24 hours at 37, 37, and 30°C, respectively. Strain GOS2R (pSSB 103R) was cultivated in two test tubes containing 5 ml of the seed culture medium as described in Example 8-(2) at 30°C for 24 hours. Four ml of GOS2R (pSSB 103R) seed cultures and 3.5 ml of growth factor supplier seed culture were inoculated to a 500-ml of Erlenmeyer-flask containing 50 ml of the production medium for mixed culture fermentations consisting of 8% D-sorbitol, 0.01 % MgSO₄·7H₂O, 1% corn steep liquor, 0.1% KH₂PO₄, 0.6% CaCO₃, 1.5% urea (sterilized separately) and antifoam (one drop per flask) (pH 7.0 before sterilization) and the flask was shaken at 30°C for 46.5 hours. As a result, mixed culture with *B. megaterium* DSM No. 4026, *E. coli* HB101 and *P. putida* ATCC 21812 produced 49.9, 54.1, 31.3 g/l 2KGA, respectively.

### (2) Mixed culture fermentation of GOS2R (pSSAP-B) with E. coli in flask.

Mixed culture fermentations by GOS2R (pSSAP-B) with *E. coli* was performed in the same manner as described above except for the seed culture medium for *E. coli* containing 2% D-sorbitol instead of 2% L-sorbose. From 10% of D-sorbitol, GOS2R (pSSAP-B) produced 73.7 g/12KGA in 48.5 hours.

### Example 11. 2KGA production by recombinant AADH

A reaction mixture containing 1.7 mg/ml of purified Enzyme A (purified according to Example 4), 50 mM Tris-HCl, pH 7.5, 5 mM CaCl₂, 8 mg/ml bovine serum albumine (BSA), 1mM PMS, 20 µg/ml PQQ, and 4% L-sorbose was incubated at 30°C with gentle shaking for 20 hours. As a result, about 2 g/l 2KGA (TLC assay) was produced.

The other reaction mixture containing 2.4 mg/ml each of purified Enzyme A and Enzyme B (purified according to Example 4), 50 mM Tris-HCl, pH7.5, 5 mM CaCl₂, 8 mg/ml BSA, 1 mM PMS, 20 µg/ml PQQ, and 2% D-sorbitol was incubated at 30°C with gentle shaking for 20 hours. As a result, 0.25 g/12KGA (HPLC assay) and about 5 g/l L-sorbose (TLC assay) were produced.

### Example 12. Production of aldehydes from alcohos, ketones from alcohols or carboxylic aicds and carboxylic acids from aldehydes.

Enzyme reactions with purified Enzyme A or Enzyem B and various substrates were performed as described in Example 11. The resulting products were identified by TLC and/or HPLC as shown in Table 10.

**Table 10**

| Enzyme | Substrate | Product |
|---|---|---|
| Enzyme A | D-Sorbitol | D-Glucose, L-Gulose |
| | L-Sorbose | L-Sorbosone, 2KGA |
| | L-Sorbosone | 2KGA |
| | D-Mannitol | D-Mannose |
| | D-Fructose | 2KD |
| Enzyme B | D-Glucose | D-Gluconic acid |
| | D-Sorbitol | L-Sorbose |
| | L-Sorbosone | 2KGA |
| | D-Mannitol | D-Fructose |
| | L-Idose | L-Idonic acid |
| | Glycerol | Dihydroxyacetone |
| | D-Gluconic acid | 5-Keto-D-gluconic acid |
| | D-Mannoic acid | 5-Keto-D-mannoic acid |

Enzyme A converted D-fructose to 2KD; this means that D-glucosone was also a product formed from D-fructose as the intermediate.

### Example 13. 2KGA and L-sorbose production by a transconjugant of P. putida

A resting cell mixture (2 ml) containing 1% CaCO₃, 0.3% NaCl, 1mM PMS, 5 µg/ml PQQ, 2% L-sorbose and 10 OD600 unit-cells of nalidixic acid resistant (Nal^{r}) *P. putida* [ATCC 21812] carrying pSSA102R or pVK100 was incubated at 30°C with gentle shaking for 17 hours. As a result, Nal^{r} *P. putida* [ATCC 21812] carrying pSSA102R or pVK100 produced 18.9 or 0.0 g/l of 2KGA, respectively.

A resting cell mixture (2 ml) containing 1% CaCO₃,0.3% NaCl, 1mM PMS, 5 µg/ml PQQ, 2% D-sorbitol and 10 OD600 unit-cells of Nal^{r} *P. putida* [ATCC 21812] with pSSB 103R or with pVK100 was incubated at 30°C with gentle shaking for 17 hours. As a result, Nal^{r} *P. putida* [ATCC 21812] carrying pSSB 103R or with pVK100 produced 7.8 or 0.0 g/l of L-sorbose, respectively.

### SEQUENCE LISTING

(1) GENERAL INFORMATION
   (i) APPLICANT
      NAME: F. HOFFMANN-LA ROCHE AG
      STREET: Grenzacherstrasse 124
      CITY: Basle
      COUNTRY: Switzerland
      POSTAL CODE: CH-4002
      TELEPHONE: 061- 688 25 05
      FAX: 061 - 688 13 95
      TELEX: 962292/965542 hlr c
   (ii) TITLE OF INVENTION:
      Novel Alcohol/Aldehyde Dehydrogenases
   (iii) NUMBER OF SEQUENCES: 12
   (iv) COMPUTER READABLE FORM:
      (A) MEDIUM TYPE: Floppy disk
      (B) COMPUTER: Macintosh
      (C) OPERATING SYSTEM:
      (D) SOFTWARE: MS word ver 5.1
(2) INFORMATION FOR SEQ ID NO:1:
   (i) SEQUENCE CHARACTERISTICS:
      (A) LENGTH: 1740 base pairs
      (B) TYPE: nucleic acid
      (C) STRANDEDNESS: double
      (D) TOPOLOGY: linear
   (ii) MOLECULE TYPE: DNA (genomic)
   (iii) ORIGINAL SOURCE:
      ORGANISM: *Gluconobacter oxydans*
      STRAIN: DSM 4025
   (iv) FEATURE:
      FEATURE KEY: CDS
      POSITIOIN: 1..1737
      SEQUENCING METHOD: E 
ATGAAACCGA CTTCGCTGCT TTGGGCCAGT GCTGGCGCAC TTGCATTGCT 50
TGCCGCACCC GCCTTTGCTC AAGTGACCCC CGTCACCGAT GAATTGCTGG 100
CGAACCCGCC CGCTGGTGAA TGGATCAGCT ACGGTCAGAA CCAAGAAAAC 150
TACCGTCACT CGCCCCTGAC GCAGATCACG ACTGAGAACG TCGGCCAACT 200
GCAACTGGTC TGGGCGCGCG GCATGCAGCC GGGCAAAGTC CAAGTCACGC 250
CCCTGATCCA TGACGGCGTC ATGTATCTGG CAAACCCGGG CGACGTGATC 300
CAGGCCATCG ACGCCAAAAC TGGCGATCTG ATCTGGGAAC ACCGCCGCCA 350
ACTGCCGAAC ATCGCCACGC TGAACAGCTT TGGCGAGCCG ACCCGCGGCA 400
TGGCGCTGTA CGGCACCAAC GTTTACTTTG TTTCGTGGGA CAACCACCTG 450
GTCGCCCTCG ACACCGCAAC TGGCCAAGTG ACGTTCGACG TCGACCGCGG 500
CCAAGGCGAA GACATGGTTT CGAACTCGTC GGGCCCGATC GTGGCAAACG 550
GCGTGATCGT TGCCGGTTCG ACCTGCCAAT ACTCGCCGTT CGGCTGCTTT 600
GTCTCGGGCC ACGACTCGGC CACCGGTGAA GAGCTGTGGC GCAACTACTT 650
CATCCCGCGC GCTGGCGAAG AGGGTGATGA GACTTGGGGC AACGATTACG 700
AAGCCCGTTG GATGACCGGT GCCTGGGGCC AGATCACCTA TGACCCCGTC 750
ACCAACCTTG TCCACTACGG CTCGACCGCT GTGGGTCCGG CGTCGGAAAC 800
CCAACGCGGC ACCCCGGGCG GCACGCTGTA CGGCACGAAC ACCCGTTTCG 850
CGGTGCGTCC TGACACGGGC GAGATTGTCT GGCGTCACCA GACCCTGCCC 900
 
CGCGACAACT GGGACCAGGA ATGCACGTTC GAGATGATGG TCACCAATGT 950

GGATGTCCAA CCCTCGACCG AGATGGAAGG TCTGCAGTCG ATCAACCCGA 1000

ACGCCGCAAC TGGCGAGCGT CGCGTGCTGA CCGGCGTTCC GTGCAAAACC 1050

GGCACCATGT GGCAGTTCGA CGCCGAAACC GGCGAATTCC TGTGGGCCCG 1100

TGATACCAAC TACCAGAACA TGATCGAATC CATCGACGAA AACGGCATCG 1150

TGACCGTGAA CGAAGATGCG ATCCTGAAGG AACTGGATGT TGAATATGAC 1200

GTCTGCCCGA CCTTCTTGGG CGGCCGCGAC TGGCCGTCGG CCGCACTGAA 1250

CCCCGACAGC GGCATCTACT TCATCCCGCT GAACAACGTC TGCTATGACA 1300

TGATGGCCGT CGATCAGGAA TTCACCTCGA TGGACGTCTA TAACACCAGC 1350

AACGTGACCA AGCTGCCGCC CGGCAAGGAT ATGATCGGTC GTATTGACGC 1400

GATCGACATC AGCACGGGTC GTACGCTGTG GTCGGTCGAA CGTGCTGCGG 1450

CGAACTATTC GCCCGTCTTG TCGACCGGCG GCGGCGTTCT GTTCAACGGT 1500

GGTACGGATC GTTACTTCCG CGCCCTCAGC CAAGAAACCG GCGAGACCCT 1550

GTGGCAGACC CGCCTTGCAA CCGTCGCGTC GGGCCAGGCC ATCTCTTACG 1600

AGGTTGACGG CATGCAATAT GTCGCCATCG CAGGTGGTGG TGTCAGCTAT 1650

GGCTCGGGCC TGAACTCGGC ACTGGCTGGC GAGCGAGTCG ACTCGACCGC 1700

CATCGGTAAC GCCGTCTACG TCTTCGCCCT GCCGCAATAA 1740
INFORMATION FOR SEQ ID NO:2:
   (i) SEQUENCE CHARACTERISTICS:
      (A) LENGTH: 1740 base pairs
      (B) TYPE: nucleic acid
      (C) STRANDEDNESS: double
      (D) TOPOLOGY: linear
   (ii) MOLECULE TYPE: DNA (genomic)
   (iii) ORIGINAL SOURCE:
      ORGANISM: *Gluconobacter oxydans*
      STRAIN: DSM 4025
   (iv) FEATURE:
      FEATURED KEY: CDS
      POSITIOIN: 1..1737
      SEQUENCING METHOD: E 
ATGAAGACGT CGTCTTTGCT GGTTGCGAGC GTTGCCGCGC TTGCAAGCTA 50
TAGCTCCTTT GCGCTTGCTC AAGTGACCCC CGTCACCGAT GAATTGCTGG 100
CGAACCCGCC CGCTGGTGAA TGGATCAGCT ACGGTCAGAA CCAAGAAAAC 150
TACCGTCACT CGCCCCTGAC GCAGATCACG ACTGAGAACG TCGGCCAACT 200
GCAACTGGTC TGGGCGCGCG GCATGCAGCC GGGCAAAGTC CAAGTCACGC 250
CCCTGATCCA TGACGGCGTC ATGTATCTGG CAAACCCGGG CGACGTGATC 300
CAGGCCATCG ACGCCAAAAC TGGCGATCTG ATCTGGGAAC ACCGCCGCCA 350
ACTGCCGAAC ATCGCCACGC TGAACAGCTT TGGCGAGCCG ACCCGCGGCA 400
TGGCGCTGTA CGGCACCAAC GTTTACTTTG TTTCGTGGGA CAACCACCTG 450
GTCGCCCTCG ACACCGCAAC TGGCCAAGTG ACGTTCGACG TCGACCGCGG 500
CCAAGGCGAA GACATGGTTT CGAACTCGTC GGGCCCGATC GTGGCAAACG 550
GCGTGATCGT TGCCGGTTCG ACCTGCCAAT ACTCGCCGTT CGGCTGCTTT 600
GTCTCGGGCC ACGACTCGGC CACCGGTGAA GAGCTGTGGC GCAACTACTT 650
CATCCCGCGC GCTGGCGAAG AGGGTGATGA GACTTGGGGC AACGATTACG 700
AAGCCCGTTG GATGACCGGC GTCTGGGGTC AGATCACCTA TGACCCCGTT 750
GGCGGCCTTG TCCACTACGG CTCGTCGGCT GTTGGCCCGG CTTCGGAAAC 800
CCAGCGCGGC ACCACCGGCG GCACCATGTA CGGCACCAAC ACCCGTTTCG 850
CTGTCCGTCC CGAGACTGGC GAGATCGTCT GGCGTCACCA AACTCTGCCC 900
 
CGCGACAACT GGGACCAAGA GTGCACCTTC GAGATGATGG TTGCCAACGT 950
TGACGTGCAG CCCGCAGCTG ACATGGACGG CGTCCGCTCG ATCAACCCGA 1000
ACGCCGCCAC CGGCGAGCGT CGCGTTCTGA CCGGCGTTCC GTGCAAAACC 1050
GGCACCATGT GGCAGTTCGA CGCCGAAACC GGCGAATTCC TGTGGGCCCG 1100
TGACACCAGC TACGAGAACA TCATCGAATC GATCGACGAA AACGGCATCG 1150
TGACCGTCGA CGAGTCGAAA GTTCTGACCG AGCTGGACAC CCCCTATGAC 1200
GTCTGCCCGC TGCTGCTGGG TGGCCGTGAC TGGCCGTCGG CTGCGCTGAA 1250
CCCCGATACC GGCATCTACT TTATCCCGCT GAACAACACC TGCATGGATA 1300
TCGAAGCTGT CGACCAGGAA TTCAGCTCGC TGGACGTGTA CAACCAAAGC 1350
CTGACCGCCA AAATGGCACC GGGTAAAGAG CTGGTTGGCC GTATCGACGC 1400
CATCGACATC AGCACAGGCC GCACCCTGTG GACCGCTGAG CGCGAAGCCT 1450
CGAACTACGC GCCTGTCCTG TCGACCGCTG GCGGCGTTCT GTTCAACGGC 1500
GGCACCGACC GTTACTTCCG CGCTCTCAGC CAAGAGACCG GCGAGACCCT 1550
GTGGCAGACC CGTCTGGCGA CTGTCGCTTC GGGCCAAGCT GTCTCGTACG 1600
AGATCGACGG CGTCCAATAC ATCGCCATCG GCGGCGGCGG CACGACCTAT 1650
GGTTCGTTCC ACAACCGTCC CCTGGCCGAG CCGGTCGACT CGACCGCGAT 1700
CGGTAATGCG ATGTACGTCT TCGCGCTGCC CCAGCAATAA 1740
INFORMATION FOR SEQ ID NO:3:
   (i) SEQUENCE CHARACTERISTICS:
      (A) LENGTH: 1737 base pairs
      (B) TYPE: nucleic acid
      (C) STRANDEDNESS: double
      (D) TOPOLOGY: linear
   (ii) MOLECULE TYPE: DNA (genomic)
   (iii) ORIGINAL SOURCE:
      ORGANISM: *Gluconobacter oxydans*
      STRAIN: DSM 4025
   (iv) FEATURE:
      FEATURE KEY: CDS
      POSITIOIN: 1..1734
      SEQUENCING METHOD: E 
ATGAAACTGA CGACCCTGCT GCAAAGCAGC GCCGCCCTGC TTGTGCTTGG 50
CACCATTCCC GCCCTTGCCC AAACCGCCAT CACCGATGAA ATGCTGGCGA 100
ACCCGCCCGC TGGTGAATGG ATCAACTACG GTCAGAACCA AGAGAACTAC 150
CGCCACTCGC CCCTGACGCA GATTACCGCA GACAACGTCG GCCAACTGCA 200
ACTGGTCTGG GCGCGCGGTA TGGAAGCGGG CAAGATCCAA GTGACCCCGC 250
TTGTCCATGA CGGCGTCATG TATCTGGCAA ACCCCGGTGA CGTGATCCAG 300
GCCATCGACG CCGCGACCGG CGATCTGATC TGGGAACACC GCCGCCAACT 350
GCCGAACATC GCCACGCTGA ACAGCTTTGG TGAGCCGACC CGCGGCATGG 400
CCCTCTATGG CACCAACGTC TATTTCGTCT CGTGGGACAA CCACTTGGTC 450
GCGCTGGACA CCTCGACCGG CCAAGTCGTA TTCGACGTCG ATCGCGGTCA 500
AGGCACGGAT ATGGTCTCGA ACTCGTCCGG CCCGATTGTC GCCAATGGCG 550
TCATCGTTGC GGGCTCGACC TGTCAGTATT CGCCGTTCGG CTGTTTCGTT 600
TCGGGCCACG ACTCGGCCAC CGGTGAAGAG CTGTGGCGCA ACAACTTTAT 650
CCCGCGCGCC GGCGAAGAGG GTGATGAGAC CTGGGGCAAT GATTACGAGG 700
CCCGCTGGAT GACCGGCGTT TGGGGCCAGA TCACCTATGA CCCCGTTGGC 750
GGCCTTGTCC ACTACGGCAC CTCAGCAGTT GGCCCTGCGG CCGAGATTCA 800
GCGCGGCACC GTTGGCGGCT CGATGTATGG CACCAACACC CGCTTTGCTG 850
TCCGCCCCGA GACCGGCGAG ATCGTCTGGC GTCACCAAAC TCTGCCCCGC 900
 
GACAACTGGG ACCAAGAGTG TACGTTCGAG ATGATGGTCG TCAACGTCGA 950
CGTCCAGCCC TCGGCTGAGA TGGAAGGCCT GCACGCCATC AACCCCGATG 1000
CCGCCACGGG CGAGCGTCGC GTTGTGACCG GCGTTCCGTG CAAGAACGGC 1050
ACCATGTGGC AGTTCGACGC CGAAACCGGC GAATTCCTGT GGGCGCGCGA 1100
CACCAGCTAT CAGAACCTGA TCGAAAGCGT CGATCCCGAT GGTCTGGTGC 1150
ATGTGAACGA AGATCTGGTC GTGACCGAGC TGGAAGTGGC CTATGAAATC 1200
TGCCCGACCT TCCTGGGTGG CCGCGACTGG CCGTCGGCTG CGCTGAACCC 1250
CGATACTGGC ATCTATTTCA TCCCGCTGAA CAACGCCTGT AGCGGTATGA 1300
CGGCTGTCGA CCAAGAGTTC AGCTCGCTCG ATGTGTATAA CGTCAGCCTC 1350
GACTATAAAC TGTCGCCCGG TTCGGAAAAC ATGGGCCGTA TCGACGCCAT 1400
CGACATCAGC ACCGGCCGCA CGCTGTGGTC GGCTGAACGC TACGCCTCGA 1450
ACTACGCGCC TGTCCTGTCC ACCGGCGGCG GCGTGCTGTT CAACGGCGGC 1500
ACCGACCGTT ACTTCCGCGC CCTCAGCCAA GAGACCGGCG AGACGCTGTG 1550
GCAGACCCGT CTGGCGACTG TCGCCTCGGG TCAAGCGATT TCCTATGAGA 1600
TCGACGGCGT GCAATATGTC GCCATCGGGC GCGGCGGCAC CAGCTATGGC 1650
AGCAACCACA ACCGCGCCCT GACCGAGCGG ATCGACTCGA CCGCCATCGG 1700
CAGCGCGATC TATGTCTTTG CTCTGCCGCA GCAGTAA 1737
INFORMATION FOR SEQ ID NO:4:
   (i) SEQUENCE CHARACTERISTICS:
      (A) LENGTH: 1740 base pairs
      (B) TYPE: nucleic acid
      (C) STRANDEDNESS: double
      (D) TOPOLOGY: linear
   (ii) MOLECULE TYPE: DNA (genomic)
   (iii) ORIGINAL SOURCE:
      ORGANISM: *Gluconobacter oxydans*
      STRAIN: DSM 4025
   (iv) FEATURE:
      FEATURE KEY: CDS
      POSITIOIN: 1..1737
      SEQUENCING METHOD: E 
ATGAACCCCA CAACGCTGCT TCGCACCAGC GCGGCCGTGC TATTGCTTAC 50
CGCGCCCGCC GCATTCGCGC AGGTAACCCC GATTACCGAT GAACTGCTGG 100
CGAACCCGCC CGCTGGTGAA TGGATTAACT ACGGCCGCAA CCAAGAAAAC 150
TATCGCCACT CGCCCCTGAC CCAGATCACT GCCGACAACG TTGGTCAGTT 200
GCAACTGGTC TGGGCCCGCG GGATGGAGGC GGGGGCCGTA CAGGTCACGC 250
CGATGATCCA TGATGGCGTG ATGTATCTGG CAAACCCCGG TGATGTGATC 300
CAGGCGCTGG ATGCGCAAAC AGGCGATCTG ATCTGGGAAC ACCGCCGCCA 350
ACTGCCCGCC GTCGCCACGC TAAACGCCCA AGGCGACCGC AAGCGCGGCG 400
TCGCCCTTTA CGGCACGAGC CTCTATTTCA GCTCATGGGA CAACCATCTG 450
ATCGCGCTGG ATATGGAGAC GGGCCAGGTC GTATTCGATG TCGAACGTGG 500
ATCGGGCGAA GACGGCTTGA CCAGTAACAC CACGGGGCCG ATTGTCGCCA 550
ATGGCGTCAT CGTCGCGGGT TCCACCTGCC AATATTCGCC CTATGGATGC 600
TTTATCTCGG GGCACGATTC CGCGACGGGT GAGGAGCTGT GGCGCAACCA 650
CTTTATCCCG CAGCCGGGCG AAGAGGGTGA CGAGACTTGG GGCAATGATT 700
TCGAGGCGCG CTGGATGACC GGCGTCTGGG GTCAGATCAC CTATGATCCC 750
GTGACGAACC TTGTGTTCTA TGGCTCGACC GGCGTGGGCC CAGCGTCCGA 800
AACCCAGCGC GGCACGCCGG GCGGCACGCT GTATGGCACC AACACCCGCT 850
 
TTGCGGTGCG TCCCGACACG GGCGAGATTG TCTGGCGTCA CCAGACCCTG 900
CCGCGCGACA ACTGGGACCA AGAATGCACG TTCGAGATGA TGGTCGCCAA 950
CGTCGATGTG CAACCCTCGG CCGAGATGGA GGGTCTGCGC GCCATCAACC 1000
CCAATGCGGC GACGGGCGAG CGCCGTGTGC TGACGGGTGC GCCTTGCAAG 1050
ACCGGCACGA TGTGGTCGTT TGATGCGGCC TCGGGCGAAT TCCTGTGGGC 1100
GCGTGATACC AACTACACCA ATATGATCGC CTCGATCGAC GAGACCGGCC 1150
TTGTGACGGT GAACGAGGAT GCGGTGCTGA AAGAGCTGGA CGTTGAATAT 1200
GACGTCTGCC CGACCTTCCT GGGTGGGCGC GACTGGTCGT CAGCCGCACT 1250
GAACCCGGAC ACCGGCATTT ACTTCTTGCC GCTGAACAAT GCCTGCTACG 1300
ATATTATGGC CGTTGATCAA GAGTTTAGCG CGCTCGACGT CTATAACACC 1350
AGCGCGACCG CAAAACTCGC GCCGGGCTTT GAAAATATGG GCCGCATCGA 1400
CGCGATTGAT ATCAGCACCG GGCGCACCTT GTGGTCGGCG GAGCGCCCTG 1450
CGGCGAACTA CTCGCCCGTT TTGTCGACGG CAGGCGGTGT GGTGTTCAAC 1500
GGCGGGACCG ACCGCTATTT CCGTGCCCTC AGCCAGGAAA CCGGCGAGAC 1550
TTTGTGGCAG GCCCGTCTTG CGACGGTCGC GACGGGGCAG GCGATCAGCT 1600
ACGAGTTGGA CGGCGTGCAA TATATCGCCA TCGGTGCGGG CGGTCTGACC 1650
TATGGCACGC AATTGAACGC GCCGCTGGCC GAGGCAATCG ATTCGACCTC 1700
GGTCGGTAAT GCGATCTATG TCTTTGCACT GCCGCAGTAA 1740
INFORMATION FOR SEQ ID NO:5:
   (i) SEQUENCE CHARACTERISTICS:
      (A) LENGTH: 579 residues
      (B) TYPE: amino acid
      (C) TOPOLOGY: linear
   (ii) MOLECULE TYPE: protein
   (iii) ORIGINAL SOURCE:
      ORGANISM: *Gluconobacter oxydans*
      STRAIN: DSM 4025
   (iv) FEATURE:
      FEATURE KEY: sig peptide
      POSITION: -23..-1
      SEQUENCING METHOD: E
      FEATURE KEY: mat peptide
      POSITION: 1..556
      SEQUENCING METHOD: E 
Met Lys Pro Thr Ser Leu Leu Trp Ala Ser Ala Gly Ala Leu Ala
          -20                 -15                 -10

Leu Leu Ala Ala Pro Ala Phe Ala Gln Val Thr Pro Val Thr Asp
          -5                  1               5

Glu Leu Leu Ala Asn Pro Pro Ala Gly Glu Trp Ile Ser Tyr Gly
        10                  15                  20

Gln Asn Gln Glu Asn Tyr Arg His Ser Pro Leu Thr Gln Ile Thr
        25                  30                  35

Thr Glu Asn Val Gly Gln Leu Gln Leu Val Trp Ala Arg Gly Met
        40                  45                  50

Gln Pro Gly Lys Val Gln Val Thr Pro Leu Ile His Asp Gly Val
        55                  60                  65

Met Tyr Leu Ala Asn Pro Gly Asp Val Ile Gln Ala Ile Asp Ala
        70                  75                  80

Lys Thr Gly Asp Leu Ile Trp Glu His Arg Arg Gln Leu Pro Asn
        85                  90                  95
 
Ile Ala Thr Leu Asn Ser Phe Gly Glu Pro Thr Arg Gly Met Ala
100 105 110

Leu Tyr Gly Thr Asn Val Tyr Phe Val Ser Trp Asp Asn His Leu
115 120 125

Val Ala Leu Asp Thr Ala Thr Gly Gln Val Thr Phe Asp Val Asp
130 135 140

Arg Gly Gln Gly Glu Asp Met Val Ser Asn Ser Ser Gly Pro Ile
145 150 155

Val Ala Asn Gly Val Ile Val Ala Gly Ser Thr Cys Gln Tyr Ser
160 165 170

Pro Phe Gly Cys Phe Val Ser Gly His Asp Ser Ala Thr Gly Glu
175 180 185

Glu Leu Trp Arg Asn Tyr Phe Ile Pro Arg Ala Gly Glu Glu Gly
190 195 200

Asp Glu Thr Trp Gly Asn Asp Tyr Glu Ala Arg Trp Met Thr Gly
205 210 215

Ala Trp Gly Gln Ile Thr Tyr Asp Pro Val Thr Asn Leu Val His
220 225 230

Tyr Gly Ser Thr Ala Val Gly Pro Ala Ser Glu Thr Gln Arg Gly
235 240 245

Thr Pro Gly Gly Thr Leu Tyr Gly Thr Asn Thr Arg Phe Ala Val
250 255 260

Arg Pro Asp Thr Gly Glu Ile Val Trp Arg His Gln Thr Leu Pro
265 270 275

Arg Asp Asn Trp Asp Gln Glu Cys Thr Phe Glu Met Met Val Thr
280 285 290

Asn Val Asp Val Gln Pro Ser Thr Glu Met Glu Gly Leu Gln Ser
295 300 305

Ile Asn Pro Asn Ala Ala Thr Gly Glu Arg Arg Val Leu Thr Gly
310 315 320

Val Pro Cys Lys Thr Gly Thr Met Trp Gln Phe Asp Ala Glu Thr
325 330 335

Gly Glu Phe Leu Trp Ala Arg Asp Thr Asn Tyr Gln Asn Met Ile
340 345 350

Glu Ser Ile Asp Glu Asn Gly Ile Val Thr Val Asn Glu Asp Ala
355 360 365

Ile Leu Lys Glu Leu Asp Val Glu Tyr Asp Val Cys Pro Thr Phe
370 375 380
 
Leu Gly Gly Arg Asp Trp Pro Ser Ala Ala Leu Asn Pro Asp Ser
        385                 390                 395

Gly Ile Tyr Phe Ile Pro Leu Asn Asn Val Cys Tyr Asp Met Met
        400                 405                 410

Ala Val Asp Gln Glu Phe Thr Ser Met Asp Val Tyr Asn Thr Ser
        415                 420                 425

Asn Val Thr Lys Leu Pro Pro Gly Lys Asp Met Ile Gly Arg Ile
        430                 435                 440

Asp Ala Ile Asp Ile Ser Thr Gly Arg Thr Leu Trp Ser Val Glu
        445                 450                 455

Arg Ala Ala Ala Asn Tyr Ser Pro Val Leu Ser Thr Gly Gly Gly
        460                 465                 470

Val Leu Phe Asn Gly Gly Thr Asp Arg Tyr Phe Arg Ala Leu Ser
        475                 480                 485

Gln Glu Thr Gly Glu Thr Leu Trp Gln Thr Arg Leu Ala Thr Val
        490                 495                 500

Ala Ser Gly Gln Ala Ile Ser Tyr Glu Val Asp Gly Met Gln Tyr
        505                 510                 515

Val Ala Ile Ala Gly Gly Gly Val Ser Tyr Gly Ser Gly Leu Asn
        520                 525                 530

Ser Ala Leu Ala Gly Glu Arg Val Asp Ser Thr Ala Ile Gly Asn
        535                 540                 545

Ala Val Tyr Val Phe Ala Leu Pro Gln
        550                 555
INFORMATION FOR SEQ ID NO:6:
   (i) SEQUENCE CHARACTERISTICS:
      (A) LENGTH: 579 residues
      (B) TYPE: amino acid
      (C) TOPOLOGY: linear
   (ii) MOLECULE TYPE: protein
   (iii) ORIGINAL SOURCE:
      ORGANISM: *Gluconobacter oxydans*
      STRAIN: DSM 4025
   (iv) FEATURE:
      FEATURE KEY: sig peptide
      POSITION: -23..-1
      SEQUENCING METHOD: S
      FEATURE KEY: mat peptide
      POSITION: 1..556
      SEQUENCING METHOD: S 
Met Lys Thr Ser Ser Leu Leu Val Ala Ser Val Ala Ala Leu Ala
-20 -15 -10
Ser Tyr Ser Ser Phe Ala Leu Ala Gln Val Thr Pro Val Thr Asp
-5 1 5
Glu Leu Leu Ala Asn Pro Pro Ala Gly Glu Trp Ile Ser Tyr Gly
10 15 20
Gln Asn Gln Glu Asn Tyr Arg His Ser Pro Leu Thr Gln Ile Thr
25 30 35
Thr Glu Asn Val Gly Gln Leu Gln Leu Val Trp Ala Arg Gly Met
40 45 50
Gln Pro Gly Lys Val Gln Val Thr Pro Leu Ile His Asp Gly Val
55 60 65
Met Tyr Leu Ala Asn Pro Gly Asp Val Ile Gln Ala Ile Asp Ala
70 75 80
 
Lys Thr Gly Asp Leu Ile Trp Glu His Arg Arg Gln Leu Pro Asn
85 90 95

Ile Ala Thr Leu Asn Ser Phe Gly Glu Pro Thr Arg Gly Met Ala
100 105 110

Leu Tyr Gly Thr Asn Val Tyr Phe Val Ser Trp Asp Asn His Leu
115 120 125

Val Ala Leu Asp Thr Ala Thr Gly Gln Val Thr Phe Asp Val Asp
130 135 140

Arg Gly Gln Gly Glu Asp Met Val Ser Asn Ser Ser Gly Pro Ile
145 150 155

Val Ala Asn Gly Val Ile Val Ala Gly Ser Thr Cys Gln Tyr Ser
160 165 170

Pro Phe Gly Cys Phe Val Ser Gly His Asp Ser Ala Thr Gly Glu
175 180 185

Glu Leu Trp Arg Asn Tyr Phe Ile Pro Arg Ala Gly Glu Glu Gly
190 195 200

Asp Glu Thr Trp Gly Asn Asp Tyr Glu Ala Arg Trp Met Thr Gly
205 210 215

Val Trp Gly Gln Ile Thr Tyr Asp Pro Val Gly Gly Leu Val His
220 225 230

Tyr Gly Ser Ser Ala Val Gly Pro Ala Ser Glu Thr Gln Arg Gly
235 240 245

Thr Thr Gly Gly Thr Met Tyr Gly Thr Asn Thr Arg Phe Ala Val
250 255 260

Arg Pro Glu Thr Gly Glu Ile Val Trp Arg His Gln Thr Leu Pro
265 270 275

Arg Asp Asn Trp Asp Gln Glu Cys Thr Phe Glu Met Met Val Ala
280 285 290

Asn Val Asp Val Gln Pro Ala Ala Asp Met Asp Gly Val Arg Ser
295 300 305

Ile Asn Pro Asn Ala Ala Thr Gly Glu Arg Arg Val Leu Thr Gly
310 315 320

Val Pro Cys Lys Thr Gly Thr Met Trp Gln Phe Asp Ala Glu Thr
325 330 335

Gly Glu Phe Leu Trp Ala Arg Asp Thr Ser Tyr Glu Asn Ile Ile
340 345 350

Glu Ser Ile Asp Glu Asn Gly Ile Val Thr Val Asp Glu Ser Lys
355 360 365
 
Val Leu Thr Glu Leu Asp Thr Pro Tyr Asp Val Cys Pro Leu Leu
        370                 375                 380

Leu Gly Gly Arg Asp Trp Pro Ser Ala Ala Leu Asn Pro Asp Thr
        385                 390                 395

Gly Ile Tyr Phe Ile Pro Leu Asn Asn Thr Cys Met Asp Ile Glu
        400                 405                 410

Ala Val Asp Gln Glu Phe Ser Ser Leu Asp Val Tyr Asn Gln Ser
        415                 420                 425

Leu Thr Ala Lys Met Ala Pro Gly Lys Glu Leu Val Gly Arg Ile
        430                 435                 440

Asp Ala Ile Asp Ile Ser Thr Gly Arg Thr Leu Trp Thr Ala Glu
        445                 450                 455

Arg Glu Ala Ser Asn Tyr Ala Pro Val Leu Ser Thr Ala Gly Gly
        460                 465                 470

Val Leu Phe Asn Gly Gly Thr Asp Arg Tyr Phe Arg Ala Leu Ser
        475                 480                 485

Gln Glu Thr Gly Glu Thr Leu Trp Gln Thr Arg Leu Ala Thr Val
        490                 495                 500

Ala Ser Gly Gln Ala Val Ser Tyr Glu Ile Asp Gly Val Gln Tyr
        505                 510                 515

Ile Ala Ile Gly Gly Gly Gly Thr Thr Tyr Gly Ser Phe His Asn
        520                 525                 530

Arg Pro Leu Ala Glu Pro Val Asp Ser Thr Ala Ile Gly Asn Ala
        535                 540                 545

Met Tyr Val Phe Ala Leu Pro Gln Gln
        550                 555
INFORMATION FOR SEQ ID NO:7:
   (i) SEQUENCE CHARACTERISTICS:
      (A) LENGTH: 578 residues
      (B) TYPE: amino acid
      (C) TOPOLOGY: linear
   (ii) MOLECULE TYPE: protein
   (iii) ORIGINAL SOURCE:
      ORGANISM: *Gluconobacter oxydans*
      STRAIN: DSM 4025
   (iv) FEATURE:
      FEATURE KEY: sig peptide
      POSITION: -23..-1
      SEQUENCING METHOD: S
      FEATURE KEY: mat peptide
      POSITION: 1..555
      SEQUENCING METHOD: S 
Met Lys Leu Thr Thr Leu Leu Gln Ser Ser Ala Ala Leu Leu Val
-20 -15 -10

Leu Gly Thr Ile Pro Ala Leu Ala Gln Thr Ala Ile Thr Asp Glu
-5 1 5

Met Leu Ala Asn Pro Pro Ala Gly Glu Trp Ile Asn Tyr Gly Gln
10 15 20

Asn Gln Glu Asn Tyr Arg His Ser Pro Leu Thr Gln Ile Thr Ala
25 30 35

Asp Asn Val Gly Gln Leu Gln Leu Val Trp Ala Arg Gly Met Glu
40 45 50

Ala Gly Lys Ile Gln Val Thr Pro Leu Val His Asp Gly Val Met
55 60 65

Tyr Leu Ala Asn Pro Gly Asp Val Ile Gln Ala Ile Asp Ala Ala
70 75 80
 
Thr Gly Asp Leu Ile Trp Glu His Arg Arg Gln Leu Pro Asn Ile
        85                  90                  95

Ala Thr Leu Asn Ser Phe Gly Glu Pro Thr Arg Gly Met Ala Leu
        100                 105                 110

Tyr Gly Thr Asn Val Tyr Phe Val Ser Trp Asp Asn His Leu Val
        115                 120                 125

Ala Leu Asp Thr Ser Thr Gly Gln Val Val Phe Asp Val Asp Arg
        130                 135                 140

Gly Gln Gly Thr Asp Met Val Ser Asn Ser Ser Gly Pro Ile Val
        145                 150                 155

Ala Asn Gly Val Ile Val Ala Gly Ser Thr Cys Gln Tyr Ser Pro
        160                 165                 170

Phe Gly Cys Phe Val Ser Gly His Asp Ser Ala Thr Gly Glu Glu
        175                 180                 185

Leu Trp Arg Asn Asn Phe Ile Pro Arg Ala Gly Glu Glu Gly Asp
        190                 195                 200

Glu Thr Trp Gly Asn Asp Tyr Glu Ala Arg Trp Met Thr Gly Val
        205                 210                 215

Trp Gly Gln Ile Thr Tyr Asp Pro Val Gly Gly Leu Val His Tyr
        220                 225                 230

Gly Thr Ser Ala Val Gly Pro Ala Ala Glu Ile Gln Arg Gly Thr
        235                 240                 245

Val Gly Gly Ser Met Tyr Gly Thr Asn Thr Arg Phe Ala Val Arg
        250                 255                 260

Pro Glu Thr Gly Glu Ile Val Trp Arg His Gln Thr Leu Pro Arg
        265                 270                 275

Asp Asn Trp Asp Gln Glu Cys Thr Phe Glu Met Met Val Val Asn
        280                 285                 290

Val Asp Val Gln Pro Ser Ala Glu Met Glu Gly Leu His Ala Ile
        295                 300                 305

Asn Pro Asp Ala Ala Thr Gly Glu Arg Arg Val Val Thr Gly Val
        310                 315                 320

Pro Cys Lys Asn Gly Thr Met Trp Gln Phe Asp Ala Glu Thr Gly
        325                 330                 335

Glu Phe Leu Trp Ala Arg Asp Thr Ser Tyr Gln Asn Leu Ile Glu
        340                 345                 350

Ser Val Asp Pro Asp Gly Leu Val His Val Asn Glu Asp Leu Val
        355                 360                 365
 
Val Thr Glu Leu Glu Val Ala Tyr Glu Ile Cys Pro Thr Phe Leu
370 375 380

Gly Gly Arg Asp Trp Pro Ser Ala Ala Leu Asn Pro Asp Thr Gly
385 390 395

Ile Tyr Phe Ile Pro Leu Asn Asn Ala Cys Ser Gly Met Thr Ala
400 405 410

Val Asp Gln Glu Phe Ser Ser Leu Asp Val Tyr Asn Val Ser Leu
415 420 425

Asp Tyr Lys Leu Ser Pro Gly Ser Glu Asn Met Gly Arg Ile Asp
430 435 440

Ala Ile Asp Ile Ser Thr Gly Arg Thr Leu Trp Ser Ala Glu Arg
445 450 455

Tyr Ala Ser Asn Tyr Ala Pro Val Leu Ser Thr Gly Gly Gly Val
460 465 470

Leu Phe Asn Gly Gly Thr Asp Arg Tyr Phe Arg Ala Leu Ser Gln
475 480 485

Glu Thr Gly Glu Thr Leu Trp Gln Thr Arg Leu Ala Thr Val Ala
490 495 500

Ser Gly Gln Ala Ile Ser Tyr Glu Ile Asp Gly Val Gln Tyr Val
505 510 515

Ala Ile Gly Arg Gly Gly Thr Ser Tyr Gly Ser Asn His Asn Arg
520 525 530

Ala Leu Thr Glu Arg Ile Asp Ser Thr Ala Ile Gly Ser Ala Ile
535 540 545

Tyr Val Phe Ala Leu Pro Gln Gln
550 555
INFORMATION FOR SEQ ID NO:8:
   (i) SEQUENCE CHARACTERISTICS:
      (A) LENGTH: 579 residues
      (B) TYPE: amino acid
      (C) TOPOLOGY: linear
   (ii) MOLECULE TYPE: protein
   (iii) ORIGINAL SOURCE:
      ORGANISM: *Gluconobacter oxydans*
      STRAIN: DSM 4025
   (iv) FEATURE:
      FEATURE KEY: sig peptide
      POSITION: -23..-1
      SEQUENCING METHOD: E
      FEATURE KEY: mat peptide
      POSITION: 1..556
      SEQUENCING METHOD: E 
Met Asn Pro Thr Thr Leu Leu Arg Thr Ser Ala Ala Val Leu Leu
-20 -15 -10
Leu Thr Ala Pro Ala Ala Phe Ala Gln Val Thr Pro Ile Thr Asp
-5 1 5
Glu Leu Leu Ala Asn Pro Pro Ala Gly Glu Trp Ile Asn Tyr Gly
10 15 20
Arg Asn Gln Glu Asn Tyr Arg His Ser Pro Leu Thr Gln Ile Thr
25 30 35
Ala Asp Asn Val Gly Gln Leu Gln Leu Val Trp Ala Arg Gly Met
40 45 50
Glu Ala Gly Ala Val Gln Val Thr Pro Met Ile His Asp Gly Val
55 60 65
Met Tyr Leu Ala Asn Pro Gly Asp Val Ile Gln Ala Leu Asp Ala
70 75 80
 
Gln Thr Gly Asp Leu Ile Trp Glu His Arg Arg Gln Leu Pro Ala
        85                  90                  95

Val Ala Thr Leu Asn Ala Gln Gly Asp Arg Lys Arg Gly Val Ala
        100                 105                 110

Leu Tyr Gly Thr Ser Leu Tyr Phe Ser Ser Trp Asp Asn His Leu
        115                 120                 125

Ile Ala Leu Asp Met Glu Thr Gly Gln Val Val Phe Asp Val Glu
        130                 135                 140

Arg Gly Ser Gly Glu Asp Gly Leu Thr Ser Asn Thr Thr Gly Pro
        145                 150                 155

Ile Val Ala Asn Gly Val Ile Val Ala Gly Ser Thr Cys Gln Tyr
        160                 165                 170

Ser Pro Tyr Gly Cys Phe Ile Ser Gly His Asp Ser Ala Thr Gly
        175                 180                 185

Glu Glu Leu Trp Arg Asn His Phe Ile Pro Gln Pro Gly Glu Glu
        190                 195                 200

Gly Asp Glu Thr Trp Gly Asn Asp Phe Glu Ala Arg Trp Met Thr
        205                 210                 215

Gly Val Trp Gly Gln Ile Thr Tyr Asp Pro Val Thr Asn Leu Val
        220                 225                 230

Phe Tyr Gly Ser Thr Gly Val Gly Pro Ala Ser Glu Thr Gln Arg
        235                 240                 245

Gly Thr Pro Gly Gly Thr Leu Tyr Gly Thr Asn Thr Arg Phe Ala
        250                 255                 260

Val Arg Pro Asp Thr Gly Glu Ile Val Trp Arg His Gln Thr Leu
        265                 270                 275

Pro Arg Asp Asn Trp Asp Gln Glu Cys Thr Phe Glu Met Met Val
        280                 285                 290

Ala Asn Val Asp Val Gln Pro Ser Ala Glu Met Glu Gly Leu Arg
        295                 300                 305

Ala Ile Asn Pro Asn Ala Ala Thr Gly Glu Arg Arg Val Leu Thr
        310                 315                 320

Gly Ala Pro Cys Lys Thr Gly Thr Met Trp Ser Phe Asp Ala Ala
        325                 330                 335

Ser Gly Glu Phe Leu Trp Ala Arg Asp Thr Asn Tyr Thr Asn Met
        340                 345                 350

Ile Ala Ser Ile Asp Glu Thr Gly Leu Val Thr Val Asn Glu Asp
        355                 360                 365
 
Ala Val Leu Lys Glu Leu Asp Val Glu Tyr Asp Val Cys Pro Thr
        370                 375                 380

Phe Leu Gly Gly Arg Asp Trp Ser Ser Ala Ala Leu Asn Pro Asp
        385                 390                 395

Thr Gly Ile Tyr Phe Leu Pro Leu Asn Asn Ala Cys Tyr Asp Ile
        400                 405                 410

Met Ala Val Asp Gln Glu Phe Ser Ala Leu Asp Val Tyr Asn Thr
        415                 420                 425

Ser Ala Thr Ala Lys Leu Ala Pro Gly Phe Glu Asn Met Gly Arg
        430                 435                 440

Ile Asp Ala Ile Asp Ile Ser Thr Gly Arg Thr Leu Trp Ser Ala
        445                 450                 455

Glu Arg Pro Ala Ala Asn Tyr Ser Pro Val Leu Ser Thr Ala Gly
        460                 465                 470

Gly Val Val Phe Asn Gly Gly Thr Asp Arg Tyr Phe Arg Ala Leu
        475                 480                 485

Ser Gln Glu Thr Gly Glu Thr Leu Trp Gln Ala Arg Leu Ala Thr
        490                 495                 500

Val Ala Thr Gly Gln Ala Ile Ser Tyr Glu Leu Asp Gly Val Gln
        505                 510                 515

Tyr Ile Ala Ile Gly Ala Gly Gly Leu Thr Tyr Gly Thr Gln Leu
        520                 525                 530

Asn Ala Pro Leu Ala Glu Ala Ile Asp Ser Thr Ser Val Gly Asn
        535                 540                 545

Ala Ile Tyr Val Phe Ala Leu Pro Gln
        550                 555
INFORMATION FOR SEQ ID NO:9:
   (i) SEQUENCE CHARACTERISTICS:
      (A) LENGTH: 82 bases
      (B) TYPE: nucleotide
      (C) TOPOLOGY: linear
   (ii) MOLECULE TYPE: DNA
   (iii) ORIGINAL SOURCE: synthetic oligonucleotide 
CATGAAAATA AAAACAGGTG CACGCATCCT CGCATTATCC GCATTAACGA 50
CGATGATGTT TTCCGCCTCG GCTCTCGCCC AG 82
INFORMATION FOR SEQ ID NO:10:
   (i) SEQUENCE CHARACTERISTICS:
      (A) LENGTH: 83 bases
      (B) TYPE: nucleotide
      (C) TOPOLOGY: linear
   (ii) MOLECULE TYPE: DNA
   (iii) ORIGINAL SOURCE: synthetic oligonucleotide 
GTTACCTGGG CGAGAGCCGA GGCGGAAAAC ATCATCGTCG TTAATGCGGA 50
TAATGCGAGG ATGCGTGCAC CTGTTTTTAT TTT 83
INFORMATION FOR SEQ ID NO:11:
   (i) SEQUENCE CHARACTERISTICS:
      (A) LENGTH: 27 residues
      (B) TYPE: amino acid
      (C) TOPOLOGY: linear
   (ii) MOLECULE TYPE: peptide
   (iii) ORIGINAL SOURCE: *E. coli*
   (iv) FEATURE:
      FEATURE KEY: sig peptide
      POSITION: 1..26
      FEATURE METHOD: S 
Met Lys Ile Lys Thr Gly Ala Arg Ile Leu Ala Leu Ser Ala Leu
1               5                   10                  15

Thr Thr Met Met Phe Ser Ala Ser Ala Leu Ala Gln
                20                  25      27
INFORMATION FOR SEQ ID NO:12:
   (i) SEQUENCE CHARACTERISTICS:
      (A) LENGTH: 27 bases
      (B) TYPE: nucleotide
      (C) TOPOLOGY: linear
   (ii) MOLECULE TYPE: DNA
   (iii) ORIGINAL SOURCE: synthetic oligonucleotide
      GTTAGCGCGG TGGATCCCCA TTGGAGG 27

## Claims

1. A DNA molecule according to SEQ ID NO: 1 or a DNA sequence which hybridizes under stringent hybridization and stringent washing conditions with said DNA molecule, said DNA encoding a polypeptide having alcohol and/or aldehyde dehydrogenase activity.

2. The DNA molecule according to claim 1, wherein the DNA molecule is present in the form of a linear or circular DNA or an insertion DNA fragment on a chromosome.

3. A recombinant enzyme preparation which comprises an enzymatic polypeptide encoded by the DNA molecule according to claims 1 or 2.

4. The recombinant enzyme preparation according to claim 3, wherein the enzymatic polypeptide is present in the form of a homodimer.

5. A recombinant expression vector comprising one or more DNA molecules as defined by any one of claims 1 or 2.

6. The recombinant expression vector according to claim 5, wherein said DNA molecule(s) is (are) functionally linked to one or more genetic control sequence(s) and are capable of expression of the enzymatic polypeptide(s) as defined in any one of claims 3 or 4 in an appropriate host cell.

7. A recombinant host cell which carries the recombinant expression vector as claimed in any one of claims 5 to 6 or one or more of DNA molecule defined by any one of claims 1 to 2.

8. The recombinant host cell according to claim 7 which is selected from the group consisting of microorganisms, a mammalian and plant cells.

9. The recombinant host cell according to claim 7 or 8, wherein the host cell is a microorganism selected from the group consisting of bacteria, such as *Escherichia coli, Pseudomonas putida, Acetobacter xylinum, Acetobacter pasteurianus, Acetobacter aceti, Acetobacter hansenii,* and *Gluconobacter oxydans.*

10. The recombinant host cell according to claim 9, wherein the host cell is *Gluconobacter oxydans* deposited under the deposit number DSM No. 4025.

11. A process for producing a recombinant enzyme preparation having an alcohol and/or aldehyde dehydrogenase activity as defined in claim 3 or 4, which comprises cultivating a recombinant host cell defined in any one of claims 7 to 10 in an appropriate culture medium and recovering said recombinant enzyme(s).

12. A process for producing an aldehyde, ketone or carboxylic acid product from a corresponding substrate which comprises converting said substrate into the product with the aid of a biochemical action of a recombinant host cell defined in any one of claims 7 to 10.

13. A process for producing 2-keto-L-gulonic acid from L-sorbose and/or D-sorbitol which comprises converting L-sorbose and/or D-sorbitol into 2-keto-L-gulonic acid with the aid , of a biochemical action of a recombinant host cell defined in any one of claims 7 to 10.

14. A process for producing an aldehyde, ketone or carboxylic acid product from a corresponding substrate which comprises incubation of a reaction mixture containing a recombinant enzyme preparation defined in any one of claims 1 or 2 and said substrate.

15. A process for producing 2-keto-L-gulonic acid which comprises incubation of a reaction mixture containing a recombinant enzyme preparation defined in any one of claims 3 or 4 and L-sorbose and/or D-sorbitol.

16. A process for the production of L-ascorbic acid from 2-keto-L-gulonic acid **characterized** therein that a process as claimed in claim 13 or 15 is effected and the 2-keto-L-gulonic acid obtained by such process is transformed into L-ascorbic acid by a method known in the state of the art.

## Patentansprüche

1. DNA-Molekül gemäss SEQ ID NO: 1 oder DNA-Sequenz, die mit dem DNA-Molekül unter stringenten Hybridisierungs- und Waschbedingungen hybridisiert, wobei die DNA für ein Polypeptid mit Alkohol- und/oder Aldehyd-Dehydrogenaseaktivität codiert.

2. DNA-Molekül gemäss Anspruch 1, das in Form einer linearen oder zirkulären DNA oder eines DNA-Insertionsfragments auf einem Chromosom vorliegt.

3. Rekombinante Enzympräparation, die ein von dem DNA-Molekül gemäss Anspruch 1 oder 2 codiertes enzymatisches Polypeptid umfaßt.

4. Rekombinante Enzympräparation gemäss Anspruch 3, wobei das enzymatische Polypeptid in Form eines Homodimers vorliegt.

5. Rekombinanter Expressionsvektor, umfassend ein oder mehrere DNA-Moleküle wie in einem der Ansprüche 1 oder 2 definiert.

6. Rekombinanter Expressionsvektor gemäss Anspruch 5, wobei das DNA-Molekül bzw. die DNA-Moleküle mit einer oder mehreren genetischen Kontrollsequenz(en) funktionell verknüpft ist bzw. sind und zur Expression des enzymatischen Polypeptids bzw. der enzymatischen Polypeptide wie in einem der Ansprüche 3 oder 4 definiert, in einer entsprechenden Wirtszelle fähig ist bzw. sind.

7. Rekombinante Wirtszelle, die den rekombinanten Expressionsvektor nach einem der Ansprüche 5 bis 6 oder ein oder mehrere DNA-Moleküle, wie in einem der Ansprüche 1 bis 2 definiert, trägt.

8. Rekombinante Wirtszelle gemäss Anspruch 7, die aus der aus Mikroorganismen, einer Säugerzelle und Pflanzenzellen bestehenden Gruppe ausgewählt ist.

9. Rekombinante Wirtszelle gemäss Anspruch 7 oder 8, wobei die Wirtszelle ein Mikroorganismus ist, ausgewählt aus der Gruppe der Bakterien, wie etwa *Escherichia coli, Pseudomonas putida, Acetobacter xylinum, Acetobacter pasteurianus, Acetobacter aceti, Acetobacter hansenii* und *Gluconobacter oxydans.*

10. Rekombinante Wirtszelle gemäss Anspruch 9, wobei es sich um unter der Hinterlegungsnummer DSM Nr. 4025 hinterlegtes *Gluconobacter oxydans* handelt.

11. Verfahren zur Herstellung einer rekombinanten Enzympräparation mit einer in Anspruch 3 oder 4 definierten Alkohol- und/oder Aldehyd-Dehydrogenaseaktivität, umfassend das Kultivieren einer in einem der Ansprüche 7 bis 10 definierten rekombinanten Wirtszelle in einem entsprechenden Kulturmedium und Gewinnen dieses rekombinanten Enzyms bzw. dieser rekombinanten Enzyme.

12. Verfahren zur Herstellung eines Aldehyd-, Keton- oder Carbonsäureprodukts aus einem entsprechenden Substrat, umfassend das Umsetzen von besagten Substrat in das Produkt mit Hilfe einer biochemischen Reaktion einer in einem der Ansprüche 7 bis 10 definierten rekombinanten Wirtszelle.

13. Verfahren zur Herstellung von 2-Keto-L-gulonsäure aus L-Sorbose und/oder D-Sorbit, umfassend das Umsetzen von L-Sorbose und/oder D-Sorbit mit Hilfe einer biochemischen Reaktion einer in einem der Ansprüche 7 bis 10 definierten rekombinanten Wirtszelle zu 2-Keto-L-gulonsäure.

14. Verfahren zur Herstellung eines Aldehyd-, Keton- oder Carbonsäureprodukts aus einem entsprechenden Substrat, umfassend die Inkubation eines Reaktionsgemisches, enthaltend eine in einem der Ansprüche 1 oder 2 definierte rekombinante Enzympräparation sowie besagtes Substrat.

15. Verfahren zur Herstellung von 2-Keto-L-gulonsäure, umfassend Inkubation eines Reaktionsgemisches, enthaltend eine in einem der Ansprüche 3 oder 4 definierte rekombinante Enzympräparation sowie L-Sorbose und/oder D-Sorbit.

16. Verfahren zur Herstellung von L-Ascorbinsäure aus 2-Keto-L-gulonsäure, **dadurch gekennzeichnet, daß** ein Verfahren nach Anspruch 13 oder 15 ausgeführt und die mit einem solchen Verfahren erhaltene 2-Keto-L-gulonsäure mit einer im Stand der Technik bekannten Methode in L-Ascorbinsäure überführt wird.

## Revendications

1. Molécule d'ADN selon SEQ ID NO: 1 ou séquence d'ADN qui s'hybride dans des conditions d'hybridation stringente et de lavage stringent avec ladite molécule d'ADN, ledit ADN codant pour un polypeptide ayant une activité alcool et/ou aldéhyde déshydrogénase.

2. Molécule d'ADN selon la revendication 1,
**caractérisée en ce que** la molécule d'ADN est présente sous la forme d'un ADN linéaire ou circulaire ou d'un fragment d'ADN d'insertion sur un chromosome.

3. Préparation d'enzyme recombinante qui comprend un polypeptide enzymatique codé par la molécule d'ADN selon les revendications 1 ou 2.

4. Préparation d'enzyme recombinante selon la revendication 3, **caractérisée en ce que** le polypeptide enzymatique est présent sous la forme d'un homodimère.

5. Vecteur d'expression recombinant comprenant une ou plusieurs molécules d'ADN telles que définies par l'une quelconque des revendications 1 ou 2.

6. Vecteur d'expression recombinant selon la revendication 5, **caractérisé en ce que** ladite ou lesdites molécules d'ADN sont liées de manière fonctionnelle à une ou plusieurs séquences de contrôle génétique et sont susceptibles d'exprimer le ou les polypeptides enzymatiques tels que définis dans l'une quelconque des revendications 3 ou 4 dans une cellule hôte appropriée.

7. Cellule hôte recombinante qui porte le vecteur d'expression recombinant selon l'une quelconque des revendications 5 ou 6 ou une ou plusieurs des molécules d'ADN définies par l'une quelconque des revendications 1 ou 2.

8. Cellule hôte recombinante selon la revendication 7, qui est choisie dans le groupe constitué des microorganismes, des cellules de mammifères et des cellules végétales.

9. Cellule hôte recombinante selon la revendication 7 ou 8, **caractérisée en ce que** la cellule hôte est un microorganisme choisi dans le groupe constitué de bactéries, telles que *Escherichia coli, Pseudomonas putida*, *Acetobacter xylinum, Acetobacter pasteurianus, Acetobacter aceti, Acetobacter hansenii* et *Gluconobacter oxydans.*

10. Cellule hôte recombinante selon la revendication 9, **caractérisée en ce que** la cellule hôte est *Gluconobacter oxydans,* déposée sous le numéro de dépôt DSM n° 4025.

11. Procédé de production d'une préparation d'enzyme recombinante ayant une activité alcool et/ou aldéhyde déshydrogénase telle que définie dans la revendication 3 ou 4, qui comprend la mise en culture d'une cellule hôte recombinante définie dans l'une quelconque des revendications 7 à 10 dans un milieu de culture approprié et la récupération de ladite ou desdites enzymes recombinantes.

12. Procédé de production d'un produit aldéhyde, cétone ou acide carboxylique à partir d'un substrat correspondant, qui comprend la transformation dudit substrat en le produit à l'aide d'une action biochimique d'une cellule hôte recombinante définie dans l'une quelconque des revendications 7 à 10.

13. Procédé de production d'acide 2-céto-L-gulonique à partir de L-sorbose et/ou de D-sorbitol, qui comprend la transformation de L-sorbose et/ou de D-sorbitol en acide 2-céto-L-gulonique à l'aide d'une action biochimique d'une cellule hôte recombinante définie dans l'une quelconque des revendications 7 à 10.

14. Procédé de production d'un produit aldéhyde, cétone ou acide carboxylique à partir d'un substrat correspondant, qui comprend l'incubation d'un mélange réactionnel contenant une préparation d'enzyme recombinante définie dans l'une quelconque des revendications 1 ou 2 et ledit substrat.

15. Procédé de production d'acide 2-céto-L-gulonique, qui comprend l'incubation d'un mélange réactionnel contenant une préparation d'enzyme recombinante définie dans l'une quelconque des revendications 3 ou 4 et du L-sorbose et/ou du D-sorbitol.

16. Procédé de production d'acide L-ascorbique à partir d'acide 2-céto-L-gulonique, **caractérisé en ce qu'**un procédé selon la revendication 13 ou 15 est réalisé et **en ce que** l'acide 2-céto-L-gulonique obtenu par un tel procédé est transformé en acide L-ascorbique par un procédé connu dans l'art.
